# EUROPEAN PATENT APPLICATION

(11) **EP 1 449 851 A1**
(43) Date of publication of application: **25.08.2004**
(21) Application number: 02795425.4
(22) Date of filing: 27.11.2002
(51) Int. Cl.: C07K 16/28, C12P 21/08, C12N 15/00, C12N 5/20, G01N 33/53, G01N 33/563, G01N 33/577

(54) **ANTI-IL13 RECEPTOR alpha 1 NEUTRALIZING ANTIBODY**

(30) Priority: 27.11.2001 JP 2001361795
(71) Applicant: MOCHIDA PHARMACEUTICAL CO., LTD., Shinjuku-ku Tokyo 160-8515 (JP)
(72) Inventor: Shirakawa, Kamon, c/o Mochida Pharm. Co.,Ltd., Tokyo 160-8515 (JP); Manabe, Tadashi, c/o Mochida Pharm.Co.,Ltd., Tokyo 160-8515 (JP)
(74) Representative: Grünecker, Kinkeldey, Stockmair & Schwanhäusser Anwaltssozietät
(86) International application number: PCT/JP2002/012393
(87) International publication number: WO 2003/046009

(57) **Abstract**

A novel anti-IL13 receptor α1 antibody having an activity of inhibiting a cell response by IL13, in particular, having an activity of inhibiting the cell response by IL13 but not inhibiting the cell response by IL4, a characteristic amino acid sequence in a variable region thereof, and a method of detecting IL13 receptor α1 in a sample using the same are provided.

## Description

### Technical Field

The present invention relates to novel antibodies against IL13 receptor α1, which have activities of inhibiting cell responses with IL13.

### Background Art

It is known that IL13 is Th-2 type cytokine and many portions thereof have the same physiological activities as IL4. As the same physiological activities as IL4, activities of inducing production of IgE and IgG4 in activated B cells, activities of inducing CD23 and MHC expressions in B cells (Punnonen J. et al., Proc. Natl. Acad. Sci. USA, Vol.90: 3730-3734 (1993), Defrance T. et al., J. Exp. Med., Vol.179: 135-143(1994)) and antiinflammatory actions on monocytes (Minty A. et al., Nature, Vol. 362: 248-250 (1993)) are known. However, IL-13 shows no activity of inducing differentiation of native T cells to Th2 as recognized in IL-4. This is supposedly because T cells express IL4 receptors but not express IL13 receptors (Zurawski G. et al., Immunol. Today, Vol. 15: 19-26 (1994)). The IL13 receptor is a heterodimer composed of two polypeptides: IL13 receptor α1 and I14 receptor α. The IL4 receptor α is essential for exerting the activity of IL13 receptor α1 to substantially bind to IL13 (Aman M. et al., J. Biol. Chem., Vol. 271: 29265 (1996)). The IL4 receptor is a heterodimer composed of two polypeptides: IL4 receptor α and γ chains. As described above, both the IL13 receptor and the IL4 receptor commonly contain a polypeptide, IL4 receptor α as their constitutional elements, so that IL4 can bind to each of the receptors to trigger a cell response. On the other hand, IL13 binds to the IL13 receptor but not bind to the IL4 receptor.

IL13 exerts its activity by binding to an IL13 receptor expressed on a target cell. Up to now, two IL13 binding units, IL13 receptor α1 and IL13 receptor α2, have been identified. Hilton et al. identified a mouse IL13 receptor α1 capable of binding to IL13 at a low affinity (Kd = 2-10 nM) (Hilton D. et al., Proc. Natl. Acad. Sci. USA, Vol. 93: 497 (1996)). In addition, human IL13 receptor α1 to be bound to IL13 with a low affinity was also identified (Aman M. et al., J. Biol. Chem., Vol. 271: 29265 (1996)). Caput et al. identified human IL13 receptor α2 to be bound to IL13 with a high affinity (Kd = 0.25 nM) (Caput D. et al., J. Biol. Chem., Vol. 271: 16921 (1996)).

As for signal transduction through the IL13 receptor, it has been reported that Tyk2 and STAT6 of Jak (Janus tyrosine kinase) family can be activated by IL13 (Izuhara K. et al., Arch. Immunol. Ther. Exp., Vol. 48: 502-512 (2000)). The IL4 receptor α associates with Jak1 and activates STAT6. The IL13 receptor α1 is a member of the type-I cytokine receptor family and has a WSXWS motif in an extracellular domain and Box 1 and Box 3 motifs retained in an intracellular domain. Box 1 and Box 3 are involved in recruiting Jak and STAT, respectively (Stahl N. et al., Science, Vol. 267; 1349-1353 (1995)). A recent study has revealed that the intracellular domain of the IL13 receptor α1 is associated with Tyk2 and STAT3 and the bindings of IL4 and IL13 causes the phosphorylation of STAT3 (Orchansky P. et al., J. Biol. Chem., Vol. 274: 20818-20825 (1999)). On the other hand, the IL13 receptor α2 cannot contribute to the signal transduction because of its short intracellular domain, so that it may function as a decoy receptor. However, a detailed function thereof has not been revealed.

As for the relationship between IL13 and diseases, evidences for involvement of IL13 in various types of allergic diseases have been piling up. There have been reported that the expression of IL13 is up-regulated in lesions and peripheral blood of patients of bronchial asthma (Huang SK. et al., J. Immunol., Vol. 155: 2688-2694 (1995), Kotsimbos TC. et al., Proc. Assoc. Am. Physicians, Vol. 108: 368-373 (1996)), atopic dermatitis (Hamid Q. et al., J. Allergy Clin. Immunol., Vol. 98: 225-231 (1996), Van der Ploeg I. et al., Clin. Exp. Immunol., Vol. 109: 526-532 (1997)), allergic rhinitis (Pawankar RU. et al., Am. J. Respir. Crit. Care Med., Vol. 152: 2059-2067 (1995), Ghaffar O. et al., Am. J. Respir. Cell. Mol. Biol., Vol. 17: 17-24 (1997)), or allergic conjunctivitis (Fujishima H. et al., Invest. Ophthalmol. Vis. Sci., Vol. 38: 1350-1357 (1997)).

The IL13 is cytokine produced by mast cells, basophils, and so on in addition to T cells. The IL13 is revealed as a control factor of strong airway contraction from mouse airway hypersensitive model and allergic asthma model. In disease of asthma, the IL13 is assumed to function to also cause an increase in airway hypersensitivity and mucous secretion from respiratory epithelium cells independent of the physiological action through IgE or eosinophils (Wills-Karp M. et al., Science, Vol. 282: 2258-2261 (1998), Grunig G. et al., Science, Vol. 282: 2261-2263 (1998)). From the test of an asthmatic crisis model obtained by introduction of OVA-sensitized helper T cells derived from an IL13 defective mouse to an IL4 receptor defective mouse, it is found that the IL13 causes an increase in airway hypersensitivity, eosinophilic infiltration, and eotaxin concentration in the lungs on the basis of unknown signals other than the signals from the IL4 receptor α (Mattes J. et al., J. Immunol., Vol. 167: 1683-1692 (2001)). From the analysis on a transgenic mouse, in which IL13 was specifically expressed on the respiratory epithelium, an increase in infiltration of mononucleosis and eosinophils, caliciform cell hyperplasia, an increase in mucus secretion from the respiratory tract, fibrosis under the respiratory tract mucosa, and so on were caused and the asthmatic symptoms including an increase in airway hypersensitivity were observed (J. Clinical Investigation vol. 103 779-788 (1999)). The IL13 receptor α1 and IL4 receptor α are strongly expressed in the respiratory epithelium cells and respiratory smooth muscle but almost no expression of the IL4 receptor γ chain is found. Therefore, it is considered that the IL13 receptor is expressed in the respiratory tissue and those expression cells are provided as IL13 targets and almost no expression of the IL4 receptor is found in the respiratory tissue (Clinicopathology vol. 49 360-364 (2001)). Therefore, it is suggested that the IL13 takes a main role as compared with the IL4 in local disease portion of asthma.

As for antibodies against IL13 receptor α1, rabbit anti-sera are prepared in WO97/15663. As for monoclonal antibodies, there are reports from Poudrier J. et al. (Eur. J. Immunol., Vol. 30: 3157-3164 (2000)), Graber P. et al. (Eur. J. Immunol., Vol. 28: 4286-4298 (1998)), and Akaiwa M. et al. (Cytokine, Vol. 13: 75-84 (2001)) but any anti-IL13 receptor α1 antibody having neutralization activity has not been yet obtained. It is reported that antibodies against IL4 receptor inhibit the responses of TF1 cells and monocytes to IL13 and IL4 (Zurawski S. et al., J. Biol. Chem., Vol. 270: 13896-13878 (1995). It is considered that the nonspecificity of inhibitory action of the antibody on the actions of IL13 and IL4 stands on the fact that the IL13 receptor and IL4 receptor share the IL4 receptor α as their structural elements.

### Disclosure of the Invention

As described above, observed is an increase in expression of IL13 in a lesion of inflammatory diseases, so that it is expected to be an effective therapeutic agent for inflammatory diseases if binding between IL13 and IL13 receptor is inhibited and an antibody for neutralizing the action of IL13 is obtained. The IL4 has the same physiological activities as the IL13 and a large quantity of IL13 is found in the inflammatory lesion and is involved in inflammatory reaction, while IL4 is suggested to assume constant roles of class switching in antibody production, Th2 differentiation of immune cells, and so on. When the IL13 and IL4 are inhibited nonselectively, an unexpected side effect may occur as the action of IL4 is inhibited in addition to a primary object to suppress the local inflammatory reaction by suppressing the action of IL13. It is conceivable that a local inflammatory reaction will be effectively inhibited without any side effect if the action of IL13 is selectively inhibited. However, anti-IL13 receptor α1 antibodies having neutralizing activities have not been yet obtained up to now. Besides, antibodies having selective neutralizing activities, which inhibit the action of IL13 but not inhibit the action of IL4 among the actions of IL13 and IL4 to the IL13 receptor, have not been yet obtained.

The present invention provides the following aspects of the present invention for solving the problems in the conventional technique.
1. An anti-IL13 receptor α1 antibody, which has an activity of inhibiting a cell response by IL13.
2. An anti-IL13 receptor α1 antibody, characterized in that the anti-IL13 receptor α1 antibody has an activity of inhibiting a cell response by IL13 but does not inhibit a cell response by IL4.
3. The anti-IL13 receptor α1 antibody according to (1) or (2), in which the anti-IL13 receptor α1 antibody is bound to an IL13 receptor α1 at a dissociation constant of at least 10 nM.
4. The anti-IL13 receptor α1 antibody according to any one of (1) to (3), in which the cell response by IL13 is a cell proliferation of TF-1 cells, and the activity of inhibiting the cell response by IL13 is at least one activity selected from the group consisting of (a-1) to (a-3) below:
   (a-1) an antibody concentration required for shifting a dose-response curve of IL13 in the cell response by IL13 toward a twice higher concentration is 10 µg/mL or less;
   (a-2) an IC50 against the cell response in the presence of IL13 at a concentration of 1 ng/mL is 10 µg/mL or less; and
   (a-3) an antibody concentration of 10 µg/mL inhibits 50% or more of the cell response in the presence of IL13 at a concentration of 1 ng/mL.
5. The anti-IL13 receptor α1 antibody according to any one of (1) to (4), in which the anti-IL13 receptor α1 antibody is a monoclonal antibody.
6. The anti-IL13 receptor α1 antibody according to any one of (1) to (5), characterized in that the anti-IL13 receptor α1 antibody has a heavy chain variable region selected from the group consisting of (a) to (c) below:
   (a) a heavy chain variable region characterized by having a first complementarity determining region consisting of an amino acid sequence described in SEQ ID NO: 1, a second complementarity determining region consisting of an amino acid sequence described in SEQ ID NO: 2, and a third complementarity determining region consisting of an amino acid sequence described in SEQ ID NO: 3;
   (b) a heavy chain variable region characterized by having a first complementarity determining region consisting of an amino acid sequence described in SEQ ID NO: 6, a second complementarity determining region consisting of an amino acid sequence described in SEQ ID NO: 7, and a third complementarity determining region consisting of an amino acid sequence described in SEQ ID NO: 8; and
   (c) a heavy chain variable region characterized by having a first complementarity determining region consisting of an amino acid sequence described in SEQ ID NO: 12, a second complementarity determining region consisting of an amino acid sequence described in SEQ ID NO: 13, and a third complementarity determining region consisting of an amino acid sequence described in SEQ ID NO: 14.
7. The anti-IL13 receptor α1 antibody according to (6), characterized by including a light chain variable region having: a first complementarity determining region consisting of one amino acid sequence selected from the group consisting of SEQ ID NO: 4, 9, and 15; a second complementarity determining region consisting of one amino acid sequence selected from the group consisting of SEQ ID NO: 5, 10, and 16; and a third complementarity determining region consisting of one amino acid sequence selected from the group consisting of SEQ ID NO: 11 and 17.
8. An antibody, which is produced by hybridoma F997-20-1, hybridoma F997-13-1, or hybridoma F997-10-1.
9. The anti-IL13 receptor α1 antibody according to any one of (1) to (7), in which the anti-IL13 receptor α1 antibody is a humanized antibody.
10. The anti-IL13 receptor α1 antibody according to (9), in which the humanized antibody includes a human chimeric antibody.
11. The antibody according to (10), in which the human chimeric antibody is a human chimeric antibody consisting of: the heavy chain variable region and the light chain variable region of the antibody according to any one of (1) to (7); and a heavy chain constant region and a light chain constant region of a human antibody.
12. The antibody according to (10), in which amino acid sequences of the heavy chain variable region and the light chain variable region are the same as amino acid sequences of a heavy chain variable region and a light chain variable region of an antibody produced by hybridoma F997-20-1, hybridoma F997-13-1, or hybridoma F997-10-1.
13. The anti-IL13 receptor α1 antibody according to (9), in which the humanized antibody includes a human complementarity determining region grafted antibody.
14. The antibody according to (13), in which the human complementarity determining region grafted antibody is a human complementarity determining region grafted antibody consisting of: the heavy chain complementarity determining region and the light chain complementarity determining region of the antibody according to any one of (1) to (7); and a heavy chain constant region, a light chain constant region, and a framework region of a human antibody.
15. The antibody according to (14), in which amino acid sequences of the heavy chain complementarity determining region and the light chain complementarity determining region have the same amino acid sequences as amino acid sequences of a heavy chain complementarity determining region and a light chain complementarity determining region of an antibody produced by hybridoma F997-20-1, hybridoma F997-13-1, or hybridoma F997-10-1.
16. The anti-IL13 receptor α1 antibody according to any one of (1) to (7), in which the anti-IL13 receptor α1 antibody is a human antibody obtained from a human antibody phage library or a human antibody-producing transgenic animal.
17. An anti-IL13 receptor α1 antibody fragment, including a part of the antibody according to any one of (1) to (8) with an activity of inhibiting a cell response by IL13.
18. A hybridoma, which produces the antibody according to any one of (1) to (7).
19. A hybridoma F997-20-1, hybridoma F997-13-1, or hybridoma F997-10-1.
20. A method of detecting an IL13 receptor α1 in a test sample using the antibody or the antibody fragment according to any one of (1) to (17).
21. A method of inhibiting an action of IL13 to an IL13 receptor-expressing cell using the antibody or the antibody fragment according to any one of (1) to (17).

### Brief Description of the Drawings

Fig. 1 is a graph that represents results of determining specificities of 13 kinds of monoclonal antibodies against IL13 R (receptor) α1 by an ELISA method on the basis of reactivities thereof with human IL13 Rα1-Fc (denoted by Fc in the figure), human IL13 Rα1-His (denoted by His in the figure), and Human IgG.
Fig. 2 is a diagram showing neutralizing activities of anti-IL13 receptor α1 antibody to the growth reaction of IL13-stimulated TF-1 cells.
Fig. 3 is a diagram showing inhibition activities of anti-IL13 receptor α1 neutralizing antibody to a cell response by IL4.
Fig. 4 is a diagram showing the inhibition activities of anti-IL13 receptor α1 neutralizing antibody to the cell response by GM-CSF.
Fig. 5 is a graph that represents standard curves created by combinations of sandwich ELISA antibodies using 6 kinds of IL13 Rα1 monoclonal antibodies.
Fig. 6 is a graph that represents standard curves of the sandwich ELISA system using a monoclonal antibody F994-7-2 and an F997-20-1 antibody.
Fig. 7 is a graph that represents results of serum measurements on normal individuals and allergic subjects using the sandwich ELISA system with the monoclonal antibody F994-7-2 and F997-20-1 antibody.
Fig. 8 is a diagram showing amino acid sequences obtained by translating the gene sequences of heavy chain and light chain variable regions of F997-10-1 antibody, F997-13-1 antibody, and F997-20-1 antibody. In the figure, the sequence surrounded by a frame shows a CDR region. In the figure, X represents an amino acid sequence which could not be translated because of an undefined base sequence thereof.

### Best Mode for carrying out the Invention

The anti-IL13 receptor α1 antibody means an antibody that specifically recognizes IL13 receptor α1. The anti-IL13 receptor α1 antibody of the present invention is characterized by inhibiting a cell response by IL13. In other words, the antibody of the present invention is an IL13 receptor α1 neutralizing antibody. The cell response by IL13 means a physiological change in cells, which occurs at the time of allowing the IL13 to act on IL13 receptor-expressing cells. The anti-IL13 receptor α1 antibody of the present invention has the activity of inhibiting and/or neutralizing the action of IL13 to the IL13 receptor-expressing cells. The IL13 receptor-expressing cells described herein may be those naturally expressing IL13 receptors among cells in the living body, cells derived from the living body, or culture cell strains, or alternatively cells expressing IL13 receptors through genetic engineering. Specific examples of IL13 receptor-expressing cells include B cells, activated B cells, monocytes, TF-1 cells, carcinoma cell lines, glioma cell lines, respiratory epithelium cells and bronchi smooth muscle cells, but not limited to them. In addition, the IL13 and the IL13 receptor may be derived from any animal species, and preferably they may be derived from the same species and used in combination. Those of human origin are particularly preferable. When the IL13 binds to the IL13 receptor (a heterodimer composed of IL13 receptor α1 and IL4 receptor α) on the cell membrane, the activation of IL13 receptor occurs. The activation of IL13 receptor leads to the phosphorylation of STAT6 and STAT3 and as a result of signal transduction a qualitative or quantitative change in gene expression, qualitative or quantitative change in expressed protein, morphological change in cells, physiological change in cells such as a change in cell proliferation occur. The "cell response by IL13" used in this specification may be any of the physical changes that have occurred in cells at the time of allowing the IL13 to act on the IL13 receptor-expressing cells as far as the change can be detected by experimental means which can be ordinarily used by a person skilled in the art. The experimental means may be preferably an in vitro assay system. Examples of the in vitro assay system will be described below.

For instance, when the IL13 is allowed to act on human erythroblast TF-1 cells (ATCC No. CRL-2003, Kitamura T. et al., Int. Immunol. Vol. 3: 571-577 (1991)), IL13-dose dependent cell proliferation as a cell response occurs. The cell proliferation can be not only detected as an increase in absorbance by the MTT assay (a method of measuring the number of living cells with a change in color by taking advantage of the fact that the living cells degrade MTT) but also detected by uptake of radioisotope-labeled thymidine. In addition, when the IL13 is allowed to act on human monocytes in the presence of CD40 ligand or CD40 agonist antibody, an increase in production of IgE as a cell response is caused (Defrance T. et al., J. Exp. Med., Vol. 179: 135-143 (1994)). The increase of IgE production can be determined by an enzyme immunoassay using anti-IgE antibody, or the like. Furthermore, when the IL13 is allowed to act on human monocytes in the presence of LPS, an increase in expression of CD23 as a cell response occurs (Zurawski S. et al., J. Biol. Chem., Vol. 270: 13869-13878 (1995)). Furthermore, when the IL13 is allowed to act on cells expressing IL13 receptor by itself such as TF-1 cells or human monocytes, phosphorylation of STAT6 occurs. The phosphorylation of STAT6 can be detected by performing PAGE after immunoprecipitation of a homogenized cell solution with anti-STAT6 antibody and then performing the Western blot method using anti-phosporylated tyrosine antibody (A. Tomkinson et al., The Journal of Immunology, Vol. 166: 5792-5800 (2001)). The antibody of the present invention may have the activity of inhibiting at least one of the cell responses with the IL13 as described above.

The inhibition of the cell response by IL13 means that the level of cell response by IL13 in the antibody-added group of the present invention is lower than that of the control group, for example the antibody-free group with no antibody of the present invention. For instance, it means a cell-response inhibition rate of 10% or more, preferably 30% or more, more preferably 50% or more, further preferably 70% or more, particularly preferably 90% or more, which is calculated from the following equation. Cell-response inhibition rate (%) = Absolute value of [(Cell response level of the control group - Cell response level of the antibody-added group) / Cell response level of the control group] ∗ 100

Here, the cell response level is appropriately defined depending on the type of cell response to be measured. For instance, when the cell response is cell proliferation, the absorbance with MTT assay can be used. When the cell response is the production of IgE, for example, the amount of IgE produced can be used. If the cell response is the expression of CD23, for example, fluorescence intensity with flow cytometry can be used.

More specifically, the neutralization activity of the antibody of the present can be represented as follows in an assay system of inhibiting IL13-dependent cell proliferation of TF-1 cells.

At an antibody concentration of 10 µg/ml, the antibody has the activity of inhibiting preferably 10% or more, more preferably 30% or more, particularly preferably 50% or more of the cell proliferation in the presence of an IL13 whose concentration is 1 ng/mL.

In other words, the inhibition of the cell response by IL13 means, when a dose-response curve of the cell response inhibition of the antibody of the present invention is obtained under the conditions of IL13 at any given concentration in an assay system of inhibiting the IL13 dependent cell proliferation of TF-1 cells, IC50 = 15 µg/mL or less, preferably 10 µg/mL or less, more preferably 7 µg/mL or less, still more preferably 3 µg/mL or less, particularly preferably 1 µg/mL. When the molecular weight of the typical IgG antibody is 150 kDa, it is equivalent to 15 µg/mL = 1 x 10⁻⁷ M and a 50% or more inhibition activity at 1 x 10⁻⁷ M may suffice for a neutralization activity.

More specifically, the antibody of the present invention preferably has the activity equal to an IC50 value of 15 µg/mL or less for the proliferation of cells in the presence of IL13 at a concentration of 1 ng/mL in the assay system of inhibiting IL13-dependent cell growth of TF-1 cells. The antibody has more preferably the activity equal to 10 µg/mL or less, particularly preferably 7µg/mL or less.

In other words, the activity of inhibiting the cell response by IL13 can be expressed as an antibody concentration required for shifting the dose-response curve of IL13 for the cell response by IL13 toward twice higher concentrations. As regards the expression in this method, if the cell response is dependent on the IL13-dose, it is effective because of allowing the characteristics within a wide dose range to be expressed in numerical terms. Furthermore, unlike a value such as an IC50 or inhibition rate varying depending on the experimental conditions in some cases, it is a numerical value of high reliability in terms of expressing an antagonist activity irrespective of the experimental conditions. The value of the antibody concentration required for shifting the dose-response curve of IL13 for the cell response by IL13 toward twice higher concentrations varies depending on the cell response to be measured. For instance, in an assay system of inhibiting the IL13-dependent cell proliferation of TF-1 cells, the antibody of the present invention has an antibody concentration of 10 µg/mL or less, preferably 7 µg/mL or less, more preferably 3 µg/mL or less, still more preferably 1.5 µg/mL or less, which is required for shifting the dose-response curve of IL13 for the cell response by IL13 toward twice higher concentrations.

For the above assay system of inhibiting the IL13-dependent cell proliferation of TF-1 cells, specifically, a method reported by Lakkis FG. et al. (Biochem Biophys Res Commun. Vol. 235: 529-532 (1997)), a method reported by Kitamura T. et al. (J. Cell Physiol. vol. 140: 323 (1989)), or the like can be suitably used in addition to a method described in Example 4.

The antibody of the present invention is characterized by having the activity of inhibiting the cell response by IL13 and the mechanism thereof can be explained as follows: it inhibits the binding between IL13 and IL13 receptor α1. However, an antibody that inhibits the binding between IL13 and IL13 receptor α1 may not always have the neutralization activity (Graber P. et al., (Eur. J. Immunol., Vol. 28: 4286-4298 (1998)). It is thought that the binding conditions between the antibody and the antigen may be determined depending on the binding strength and which site the antigen binds with the antibody in. Even if it is an antibody that inhibits the binding between IL13 and IL13 receptor α1, a neutralization activity cannot be imparted unless no appropriate binding strength and appropriate binding site (epitope) of the antigen are attained. Therefore, the anti-IL13 receptor α1 antibody of the present invention has the activity of inhibiting a cell response by IL13, and also it can be defined by determining (a) the strength of binding between the IL13 receptor α1 and the antibody of the present invention and/or (b) the site of IL13 receptor α1 where the antibody of the present invention binds to. For example, as for (a), the antibody binds to the IL13 receptor α1 at a dissociation constant (Kd) of at least 10 nM or less, preferably 5.0 nM or less, more preferably 3.0 nM or less, still more preferably 1.5 nM or less. The dissociation constant can be measured by means of surface plasmon resonance. Regarding (b), epitopes are recognized which can be recognized by antibodies produced by hybridoma F997-13-1, hybridoma F997-20-1, or hybridoma F997-10-1. The epitope can be specifically recognized by the complementarity determining region of the antibody, so that it is also possible to determine the anti-IL13 receptor α1 antibody of the present invention by the complementarity determining region of the antibody in place of the epitope. The dissociation constant based on the surface plasmon resonance can be measured, for example, by a method described in Example 2. The epitopes recognized by the antibodies produced by hybridoma F997-13-1, hybridoma F997-20-1, or hybridoma F997-10-1 on the IL13 receptor α1 can be determined by suitably preparing fragmented peptides of IL13 receptor α1 and determining whether the antibodies have reactivities to the fragments using the ELISA method or the like.

The anti-IL13 receptor α1 antibody according to the present invention is characterized by having an activity of inhibiting a cell response by IL13 and a heavy chain variable region selected from the group consisting of (a) to (c) below: (a) a heavy chain variable region characterized by having a first complementarity determining region consisting of an amino acid sequence described in SEQ ID NO: 1, a second complementarity determining region consisting of an amino acid sequence described in SEQ ID NO: 2, and a third complementarity determining region consisting of an amino acid sequence described in SEQ ID NO: 3; (b) a heavy chain variable region characterized by having a first complementarity determining region consisting of an amino acid sequence described in SEQ ID NO: 6, a second complementarity determining region consisting of an amino acid sequence described in SEQ ID NO: 7, and a third complementarity determining region consisting of an amino acid sequence described in SEQ ID NO: 8; and (c) a heavy chain variable region characterized by having a first complementarity determining region consisting of an amino acid sequence described in SEQ ID NO: 12, a second complementarity determining region consisting of an amino acid sequence described in SEQ ID NO: 13, and a third complementarity determining region consisting of an amino acid sequence described in SEQ ID NO: 14. The anti-IL13 receptor α1 antibody is
characterized by including, in addition to the heavy chain variable region, a light chain variable region having: a first complementarity determining region consisting of one amino acid sequence selected from the group consisting of amino acid sequence of SEQ ID NO: 4, amino acid sequence of SEQ ID NO: 9, and amino acid sequence of SEQ ID NO: 15; a second complementarity determining region consisting of one amino acid sequence selected from the group consisting of amino acid sequence of SEQ ID NO: 5, amino acid sequence of SEQ ID NO: 10, and amino acid sequence of SEQ ID NO: 16; and a third complementarity determining region consisting of one amino acid sequence selected from the group consisting of amino acid sequence of SEQ ID NO: 11 and amino acid sequence of SEQ ID NO: 17. Alternatively, the anti-IL13 receptor α1 antibody is characterized by including, in addition to the heavy chain variable region, as a light chain variable region, a light chain variable region selected from the group consisting of (a) to (c) below: (a) a light chain variable region characterized by having a first complementarity determining region consisting of an amino acid sequence described in SEQ ID NO: 4, and a second complementarity determining region consisting of an amino acid sequence described in SEQ ID NO: 5; (b) a light chain variable region characterized by having a first complementarity determining region consisting of an amino acid sequence described in SEQ ID NO: 9, a second complementarity determining region consisting of an amino acid sequence described in SEQ ID NO: 10, and a third complementarity determining region consisting of an amino acid sequence described in SEQ ID NO: 11; and (c) a light chain variable region characterized by having a first complementarity determining region consisting of an amino acid sequence described in SEQ ID NO: 15, a second complementarity determining region consisting of an amino acid sequence described in SEQ ID NO: 16, and a third complementarity determining region consisting of an amino acid sequence described in SEQ ID NO: 17. More preferably, the anti-IL13 receptor α1 antibody of the present invention has an activity of inhibiting a cell response by IL13 and is characterized in that the amino acid sequences in the heavy chain complementarity determining region and the light chain complementarity determining region are the same as amino acid sequences in a heavy chain complementarity determining region and a light chain complementarity determining region of an antibody, which is produced by hybridoma F997-20-1, hybridoma F997-13-1, or hybridoma F997-10-1.

Preferably the anti-IL13 receptor α1 antibody of the present invention has a feature that the antibody does not inhibit the cell response by IL14 in addition to inhibiting the cell response by IL13. The cell response by IL4 means a physiological change in IL13 receptor-expressing cells which occurs when the IL14 acts on the cells. The anti-IL13 receptor α1 antibody of the present invention has the activity of inhibiting and/or neutralizing the action of IL13 to IL13 receptor-expressing cells but does not inhibit and/or neutralize the action of IL4 to IL13 receptor-expressing cells. The phrase "does not inhibit the cell response by IL4" means that with respect to the cell response by IL4, the control group, for example, the group not added with the antibody of the present invention, is substantially equal to the antibody-added group. Specifically, it means IC50 > 15 µg/mL, more preferably IC50 > 10 µg/mL, when the dose-response curve of the cell response inhibition of the antibody of the present invention is obtained under the condition of a certain concentration of IL4.

The anti-IL13 receptor α1 antibody of the present invention may be one that recognizes the IL13 receptor α1 of any of animal species as far as it has the activity of inhibiting the cell response by IL13. Preferable is one that specifically recognizes the human IL13 receptor α1, but may have cross reactivity to mouse/human IL13 receptor α1. When it has the cross reactivity to mouse IL13 receptor α1, it is possible to examine the pharmacological activity of the antibody by administrating it to a disease model mouse, which is useful.

The anti-IL13 receptor α1 antibodies of the present invention include those with and without cross reactivity to IL13 receptor α2. However, preferable is one having no cross reactivity to IL13 receptor α2.

The cross reactivity can be suitably examined by the ELISA method in which antigen as a target of the cross reactivity test is immobilized or a method using the BIACORE system, as described in Example 2. For instance, in the method using the BIACORE system described in Example 2, it is possible to determine that there is no cross reactivity when an RU value is less than 50.

The antibodies of the present invention may be polyclonal antibodies or monoclonal antibodies. For clarifying or apparently exerting the functions of the antibodies, the monoclonal antibodies are preferable. The molecular species of antibodies of the present invention are not specifically limited. The structure of the antibodies, i.e., immunoglobulin has heavy chains (H chains) and light chains (L chains) and is classified into five isotypes (IgG, IgA, IgM, IdD, and IgE) on the basis of the respective classes (γ, α, µ, δ, and ε) of the heavy chains. Among them, IgG and IgA are further classified into subclasses (for example, for human, IgG1, IgG2, IgG3, IgG4, IgA1, and IgA2) on the basis of their different heavy chains (for example, for human, γ1, γ2, γ3, γ4, α1, and α2). The light chains can be classified into either κ or λ. The antibodies of the present invention may be antibodies which can be classified into any of classes, subclasses, or isotypes.

On the N-terminals of the heavy and light chains, there are variable regions which are called heavy chain variable regions (VH) and light chain variable regions' (VL), respectively. In the variable region, there is a complementarity determining region (CDR) responsible for the specificity of antigen recognition. A part of the variable region other than CDR functions to retain the structure of CDR and is called a frame work region (FR). On the C-terminals of the heavy and light chains, there are constant regions which are called heavy chain constant regions (CH) and light chain constant regions (CL), respectively.

In the heavy chain variable region, there are three complementarity determining regions: a first complementarity determining region (CDR1), a second complementarity determining region (CDR2), and a third complementarity determining region (CDR3). The three complementarity determining regions in the heavy chain variable region are collectively called a heavy chain complementarity determining region. Likewise, in the light chain variable region, there are a first complementarity determining region (CDR1), a second complementarity determining region (CDR2), and a third complementarity determining region (CDR3). These three complementarity determining regions in the light chain variable region are collectively called a light chain complementarity determining region.

Fragments or peptides of the anti-IL13 receptor α1 neutralizing antibody of the present invention, which have the activity of inhibiting the cell response to IL13 and contain part of the neutralizing antibody, include a fragment of antigen binding (Fab), Fab', F(ab')2, single-chain antibody (single chain Fv: scFv), disulfide stabilized antigen (disulfide stabilized Fv: dsFv), and CDR-containing peptide. Fab is the fragment of antibody having the antigen-binding activity where almost the half of amino acids on the N-terminal side and the whole of the light chain are bound through a disulfide bond, among the fragments obtained by treating IgG with a proteolytic enzyme papain.

The Fab of the present invention can be obtained by treating the anti-IL13 receptor α1 antibody of the present invention with a proteolytic enzyme papain. Alternatively, DNA encoding the Fab of the antibody is inserted into a prokaryote expression vector or eukaryote expression vector and is then expressed by introducing the vector into a prokaryote or eukaryote to produce Fab.

F(ab')2 is the fragment of antibody having antigen-binding activity, which is slightly larger than one on which Fab binds through a disulfide bond of a hinge region among the fragments obtained by treating IgG with a proteolytic enzyme papain. The F(ab')2 of the present invention can be obtained by treating the anti-IL13 receptor α1 antibody of the present invention with a proteolytic enzyme pepsin. Alternatively, it can be prepared by binding with the following Fab' through a thioether bond or disulfide bond.

Fab' is the fragment of antibody having antigen-binding activity, in which the disulfide bond of a hinge region in the above F(ab')2 is cut off. The Fab' of the present invention can be obtained by treating F(ab')2, which specifically acts on IL13 receptor α1, with a reducing agent dithiothreitol.

scFv is a polypeptide obtained by linking a VH and a VL together through an appropriate peptide linker. As the VH and VL included in the scFv of the present invention, any of antibodies produced by the hybridoma of the present invention, humanized antibodies, and human antibodies can be used. The scFv of the present invention can be produced by acquiring cDNA encoding VH and VL of the anti-IL13 receptor α1 antibody of the present invention, constructing DNA encoding scFv, inserting the DNA into a prokaryote expression vector or eukaryote expression vector, and introducing the expression vector into a prokaryote or eukaryote.

dsFV is one obtained by allowing polypeptides in which one amino acid residue in each of VH and VL is substituted with a cysteine residue to bind together through a disulfide bond between the cysteine residues. The amino acid residue substituted with the cysteine residue can be selected on the basis of conformational prediction of antibody according to the method disclosed by Reiter et al. (Protein Engineering, 7, 697 (1994)). The VH and VL included in the dsFv of the present invention can be any one of antibodies produced by the hybridoma of the present invention, humanized antibodies, and human antibodies.

The dsFv of the present invention can be produced by acquiring cDNA encoding VH and VL of the anti-IL13 receptor α1 antibody of the present invention, constructing DNA encoding dsFv, inserting the DNA into a prokaryote expression vector or eukaryote expression vector, and introducing the expression vector into a prokaryote or eukaryote.

The CDR-containing peptide is constructed by including at least one region of the H-chain CDR and the L-chain CDR. Plural CDRs can be bound together directly or through appropriate peptide linkers. The CDR-containing peptide of the present invention can be produced by acquiring cDNA encoding VH and VL of the anti-IL13 receptor α1 antibody of the present invention, constructing DNA encoding CDR, inserting the DNA into a prokaryote expression vector or eukaryote expression vector, and introducing the expression vector into a prokaryote or eukaryote. In addition, the CDR-containing peptide can be also produced by a chemical synthetic method such as the Fmoc method (fluorenylmethyloxycarbonyl method) or tBoc method (t-butyloxy carbonyl method).

The antibodies of the present invention may include any of antibodies produced by the hybridoma of the present invention, humanized antibodies, human antibodies, or antibody fragments thereof on which radioisotopes, proteins, low molecular weight compounds, or the like are coupled. They can be produced by coupling N- or C-terminals of H- or L-chains of the anti-IL13 receptor α1 antibodies or antibody fragments of the present invention, appropriate substituents in the antibody or antibody fragment or side chains, or sugar chains of the antibodies or antibody fragments with radioisotopes, proteins, low molecular weight compounds, or the like by chemical procedures ("Introduction to Antibody Engineering" (written by Osamu Kanemitsu, Chijin Shokan Co., Ltd., 1994).

The antibodies of the present invention can be produced by the technologies which are well known in the art.

An IL13 receptor α1 protein or a fragment peptide thereof is used as an antigen. An animal, from which the protein to be used as an antigen is originated, may be suitably selected depending on the application of the antibody, and a human IL13 receptor α1 protein or the fragment peptide thereof is preferable. In addition, the antigen may have activity as IL13 receptor α1 or have no such activity and may be any of an antigen of natural origin, an antigen prepared in views of genetic engineering, a chemically synthesized antigen, and an antigen provided as a fusion protein with another protein or peptide. The "activity as IL13 receptor α1" used herein means the ability of binding to IL13. The ability of binding to IL13 can be detected by the method described in Example 3, and the well-known methods such as the ELISA system that detects IL13 by immobilizing the antigen, adding the IL13 and detecting the IL13 bound to the antigen via the anti-IL13 antibody. The most preferable antigen is a fusion protein between the extracellular region of human IL13 receptor α1 and the Fc fragment of immunoglobulin. Specifically, plasmid pM1701 described in Example 1 has been deposited to the International Patent Organism Depositary, National Institute of Advanced Industrial Science and Technology, Tsukuba Central 6, 1-1 Higashi 1-chome, Tsukuba-shi, Ibaraki-ken, Japan, on November 26, 2001 (the accession number of FERM p-18632), and a preferable antigen is soluble IL13 receptor α1-Fc capable of production using (the accession number of FERM BP-8238) being transferred to the international deposition under the provision of the Budapest Treaty on November 20, 2002. Such an antigen does not have the ability of binding to IL13. However, using the antigen made it possible to acquire an antibody having the neutralization activity for the first time. It means that the antigen is one effectively providing an epitope related to the neutralization activity.

Mammals to be immunized are not specifically limited. However, a mouse, rat, hamster, or the like is preferable because it is preferable to make a selection in consideration of adaptability to myeloma cells to be used for cell fusion, in preparing a monoclonal antibody. The myeloma cells to be used may be various known cells. Examples thereof include P3, P3U1, SP2/O, NS-1, YB2/0, and Y3-Ag1, 2, 3 myeloma cells.

Immunization can be performed by the well-known methods. For instance, it can be performed by the intraperitoneal, subcutaneous, intravenous, or intra-footpad administration of the antigen. The administration of the antigen may be performed in combination with adjuvant, and the administration may be performed plural times. Preferable lymphocytes are cells originated from the lymph node or splenocytes surgically removed several days, for example three days after the final administration of the antigen.

The fusion between the lymphocytes and myeloma cells can be performed using the well-known method such as the method of Milstein et al. (Methods in Enzymol., vol. 73, p. 3). For example, it may be a method of using polyethylene glycol (PEG) as a fusion agent or an electric fusion method. The mixing ratio between the lymphocytes and myeloma cells is not limited as far as they can undergo fusion. Preferably, however, the volume of myeloma cells is equal to or one-tenth volume of the lymphocytes. In the method of using PEG (average molecular weight: 1,000-4,000) for the cell fusion, the PEG concentration is not specifically limited but the method is preferably performed at 50%. In addition, as an agent for improving the efficiency of fusion, a coadjuvant such as dimethyl sulfoxide (DMSO) may be added. The fusion is initiated by the addition of a PEG solution heated at 37°C to the mixed cells and then terminated by the addition of a culture medium after reaction for 1 to 5 minutes.

Hybridoma formed by this fusion is incubated for 1 to 7 days in a selection medium such as a medium (HAT medium) containing hypoxanthine, thymidine, and aminopterin to separate the hybridoma from unfused cells. The resulting hybridoma is further selected according to the antibodies produced therefrom. The selected hybridoma is monocloned according to the well-known limiting dilution to establish monoclonal antibody-producing hybridoma.

As a method of detecting the activity of antibodies produced by the hybridoma, any of the well known methods can be used. Here, as for the activity of antibodies, the ability of binding to IL13 receptor α1 antibody is detected as a first step and then the activity of inhibiting the cell response is detected as a second step. A method of detecting the activity in the second step is as described above. Examples of a method of detecting the activity in the first step include the ELISA method, Western blot method, and radioimmunoassay method.

The established hybridoma is incubated by the well-known method, and monoclonal antibodies can be obtained from the culture supernatant. In addition, the hybridoma is administered to mammals having adaptability to the hybridoma and is then allowed for growth therein, so that it can be obtained from the ascitic fluid thereof.

The antibodies can be purified using the well-known purification means such as a salting-out, gel filtration, ion-exchange chromatography, or affinity chromatography method.

As a method of confirming the antigen-binding ability of anti-IL13 receptor α1 antibody of the present invention or a method of detecting the IL13 receptor α1 in a biological sample using the anti-IL13 receptor α1 antibody of the present invention, there are fluorescent antibody technique, enzyme-linked immunosorbent assay (ELISA), radioimmunoassay (RIA), immunohistochemical staining techniques (e.g., ABC technique and CSA technique) such as an immunohistological staining technique and immunocytological staining technique, Western blotting method, immunoprecipitation, the enzyme immunoassay described above, and sandwich ELISA method (Monoclonal Antibody Experiment Manual (Kodansha Scientific, 1987), Biochemical Experiments Lecture Series 5: Immuno-biochemistry Research Method (Tokyo Kagaku Dojin, 1986)).

The monoclonal antibodies of the present invention include antibodies produced by hybridoma, humanized antibodies, and human antibodies. The hybridoma means cells that produce monoclonal antibodies having desired antigen specificity, which can be obtained by cell fusion between B cells acquired by immunizing the mammals except human beings with an antigen and myeloma cells originated from a mouse, rat, or the like.

The humanized antibodies include human chimeric antibodies and human CDR grafted antibodies. The human chimeric antibodies mean antibodies composed of VH and VL of the antibodies of an animal except human beings and CH and CL of the human antibodies. The human CDR grafted antibodies mean antibodies prepared by inserting the amino acid sequences of CDR for VH and VL of the antibody of an animal except human beings into the appropriate positions of VH and VL of the human antibody in terms of genetic engineering. Any of animals including a mouse, rat, hamster, and rabbit, except human beings, can be used as far as it allows the production of hybridoma.

The human chimeric antibodies and human CDR grafted antibodies of the present invention can be produced using the well known methods (for each of them, since the publication of the following: Nature, 312: 643, 1984 and Nature, 321: 522, 1986, many methods have been developed). Briefly speaking, at first, from hybridoma that produces an anti-IL13 receptor α1 monoclonal antibody having the activity of inhibiting the cell response by IL13, cDNAs that encode VH and VL are acquired and then base sequences and amino acid sequences thereof are defined. Next, in the case of the human chimeric antibody, the acquired cDNAs that encode VH and VL are inserted into animal cell expression vectors having genes encoding human antibodies CH and CL to construct human chimeric antibody expression vectors and then introduced into animal cells to express them, respectively, for the production. In the case of human CDR grafted antibody, it can be produced by constructing cDNAs that respectively encode V regions, in which CDR sequences of VH and VL of the antibody of an animal except human beings previously defined are substituted with CDR sequences of VH and VL of any human antibody, and inserting them into animal cell expression vectors respectively having genes encoding human antibodies CH and CL to construct human CDR grafted antibody expression vectors, followed by introducing the expression vectors into animal cells to express them.

As the constant regions of human antibodies used for the production of human chimeric antibody and human CDR grafted antibody, any of the constant regions of the human antibody such as Cγ1 and Cγ4 for the heavy chain constant region of the human antibody and such as Cκ for the light chain constant region of the human antibody can be used. As the amino acid sequence of a framework region of the V region in the human antibody to be used for the production of human CDR grafted antibody, any amino acid sequence can be used as far as it is the amino acid sequence of FR of the V region originated from human antibody. For instance, such an amino acid sequence may be the amino acid sequence of FR in the V region of human antibody registered in the Protein Data Bank or the common amino acid sequence for the respective subgroups of FR in the V region of human antibody (Sequences of Proteins of Immunological Interest, US Dept. Health and Human Services, 1991). For the production of human CDR grafted antibody having sufficient activity, it is desirable to have a high homology to the amino acid sequence of the V region in antibody of an animal except human beings provided as a donor of CDR, preferably a homology of 65% or more.

The constant regions or framework regions in human antibodies to be used for the production of human chimeric antibodies and human CDR grafted antibodies can be prepared according to the conventional methods (Queen et al., Proc. Natl. Acad. Sci. USA 86: 10029 (1989), WO 90/07861 and WO 92/11018, Co et al., Proc. Natl. Acad. Sci. USA, 88, 2869 (1991), Co and Queen, Nature, vol. 351, p. 501, 1991, and Co et al., J. Immunol. 148: 1149 (1992)).

The term the human antibodies generically means those naturally present in the human body and also antibodies obtained from a human antibody phage library or a human antigen-producing transgenic animal, or the like are included. The human antigen phage library is a library where antibody fragments of Fab, a single chain antibody, and the like are expressed on the surface of phage by inserting an antibody gene prepared from human B cells into a phage gene. From the library, a phage expressing antibody fragments having the desired antigen-binding activity using as an index the binding activity to a substrate having an antigen immobilized can be recovered. The antibody fragment can be also converted into a human antibody molecule having two complete H chains and two complete L chains by a genetic engineering technique.

The human antibody-producing transgenic animal can be prepared by the method developed by Ishida et al. (Proc. Natl. Acad. Sci. USA, Vol. 97: 722-727 (2000)). In other words, at first, according to the method of Ishida et al., as a trans-chromosome (Tc) mouse, human chromosome 2 fragment (Ig light chain κ) and chromosome 14 fragment (Ig heavy chain) are introduced into mouse ES cells by a microcell fusion method and then chimeric mice having the respective chromosome fragments are produced according to the method of Joyner et al. (Gene Targeting, Experimental Techniques Series, Medical Science International). Then, two kinds of the chimeric mice prepared are mated to prepare a chimeric mouse having both the human chromosome 2 fragment (Ig light chain κ) and chromosome 14 fragment (Ig heavy chain). For removing the endogenous production of mouse antibody originated from the mouse, a trans-chromosome mouse containing the human chromosome 2 fragment (Ig light chain κ) and chromosome 14 fragment (Ig heavy chain) in which the endogenous Ig heavy chain and κ chain are knocked out is prepared such that a double KO mouse in which endogenous Ig heavy chain and κ chain are knocked out according to the method of Capecchi et al. (Mol. Cell. Biol. 12: 2919-2923, 1992), and then the mouse is mated with the chimeric mouse in which the human chromosome fragments are introduced. The resulting Tc mouse produces antibodies originated from the human beings in blood but no mouse Ig heavy chain and mouse Ig light chain κ are detected. The chromosomes of the resulting Tc mouse are retained even for generations and the descendants thereof can be used for the production of anti-IL13 receptor α1 human monoclonal antibody.

50 µg of purified IL13 receptor α1 antibody is mixed with Titer Max Gold (available from CytRx, Co., Ltd.) and then subcutaneously administrated to the Tc mouse, followed by an additional administration thereof in the same way after 3 weeks. An increase in antibody titer allows the reaction of the diluted antiserum in a plate on which antigens are immobilized, and subsequently the human antibody in serum is detected using anti-human IgG antibody. The cell fusion is performed three days after administering 50 µg of antigen to the peritoneal cavity of a mouse with an increased antibody titer. Specifically, the collected splenocytes are mixed with mouse myeloma cells (SP2/O-Ag14) and then fused with PEG4000 (Merck), followed by selecting hybridomas using a HAT culture medium containing G418 (1 mg/mL). The hybridomas generated are subjected to screening using anti-human IgGκ antibody, anti-IgG antibody, anti-IgG2 antibody, anti-IgG3 antibody, or anti-IgG4 antibody as secondary antibody to select hybridoma that produces human antibody to be bound to IL13 receptor α1.

The hybridoma of the present invention is hybridoma that produces anti-IL13 receptor α1 antibody having the activity of inhibiting the cell response by IL13. The hybridoma of the present invention can be produced by the method described above. The hybridoma of the present invention is preferably hybridoma F997-20-1, hybridoma F997-13-1, or hybridoma F997-10-1.

A method of detecting IL13 receptor α1 in the test sample using the antibody or antibody fragment of the present invention may include the steps of brining the test sample into contact with the antibody or antibody fragment of the present invention and detecting the IL13 receptor α1 in the test sample bound to the antigen or antibody fragment of the present invention. The additional step of determining the quantity of IL13 receptor α1 in the test sample may be included.

The methods of detecting the IL13 receptor α1 in the test sample using the antibody of the present invention include, in addition to the sandwich ELISA system described in Examples 5 and 6, an inhibition ELISA system, fluorescent antibody method, immunohistochemical staining technique, radioimmunoassay, Western blotting method, immunoprecipitation, and so on but not limited to these methods. The target test samples are, but not limited to, biological samples, animal, in particular, human body fluid, or tissues, cells, and bacteria, and their extracts, culture supernatant, smear preparations, and sections. Among them, the body fluid is preferable. It is more preferable that a sample is selected from the blood, plasma, serum, urine, spinal fluid, lymph, saliva, and pleural fluid.

In the inhibition ELISA system, at first, an antigen-immobilized plate is prepared, followed by reaction with anti-IL13 receptor α1 monoclonal antibody as a first antibody. Simultaneously, the test sample such as culture supernatant or serum of which the IL13 receptor α1 concentration is desired to be measured is diluted and added to allow a competition reaction. The concentration of the test sample is calculated on the basis of a calibration curve prepared by gradually diluting the IL13 receptor α1 protein of the known concentration. The concentration of the subject is compared with that of the normal to determine whether the expression level is increased or not, allowing a diagnosis of whether the subject suffers from allergic disorders.

The fluorescent antibody method can be performed using the methods described in the literatures (Monoclonal Antibodies: Principles and Practice, Third Edition (Academic Press, 1996), Monoclonal Antibody Experiment Manual (Kodansha Scientific, 1987)), or the like. Specifically, the monoclonal antibody or the antibody fragment thereof of the present invention is reacted with cells or a homogenized solution thereof, a tissue or homogenized solution thereof, cell culture supernatant, serum, or the like, and then reacted with anti-immunoglobulin antibody or the binding fragment thereof labeled with a fluorescent substance such as fluorescein isothiocyanate (FITC) or phycoerythrin, followed by measuring the fluorescent dye with a flow cytometer.

The immunohistochemical staining techniques (e.g., ABC method and CSA method) such as immunocytological staining technique and immunohistological staining technique can be performed using the methods described in the literatures (Monoclonal Antibodies: Principles and Practice, Third Edition (Academic Press, 1996), Monoclonal Antibody Experiment Manual (Kodansha Scientific, 1987)). The enzyme-linked immunosorbent assay (ELISA) is a method in which the monoclonal antibody or the antibody fragment thereof of the present invention is reacted with cells or a homogenized solution thereof, a tissue or homogenized solution thereof, cell culture supernatant, serum, or the like, and then reacted with anti-immunoglobulin antibody or the binding fragment thereof labeled with an enzyme label such as peroxidase or biotin, or the like, followed by measuring the color-developing dye with an absorptiometer.

The radioimmunoassay (RIA) is a method in which the monoclonal antibody or the antibody fragment thereof of the present invention is reacted with cells or a homogenized solution thereof, a tissue or homogenized solution thereof, cell culture supernatant, serum, or the like, and then reacted with anti-immunoglobulin antibody or the binding fragment thereof labeled with a radiolabel, followed by measuring with a scintillation counter or the like.

The immunocytological staining technique and immunohistological staining technique are methods in which the monoclonal antibody or the antibody fragment thereof of the present invention is reacted with cells, tissue, or the like and then reacted with anti-immunoglobulin antibody or the binding fragment thereof labeled with a fluorescent substance such as fluorescein isothiocyanate (FITC) or subjected to an enzyme label such as peroxidase or biotin, followed by microscopic observation.

In the Western blotting method, after fractionating cells or a homogenized solution thereof, a tissue or homogenized solution thereof, cell culture supernatant, serum, or the like by an SDS-polyacrylamide gel electrophoresis (Antibodies-A Laboratory Manual, Cold Spring Harbor Laboratory,1988), the gel is blotted on a PVDF membrane or a nitrocellulose membrane. Subsequently, the monoclonal antibody or the antibody fragment thereof of the present invention is reacted with the membrane and then reacted with anti-mouse IgG antibody or the binding fragment thereof labeled with a fluorescent substance such as FITC or subjected to an enzyme label such as peroxidase or biotin, followed by confirmation.

The immunoprecipitation method is a method in which the monoclonal antibody or the antibody fragment thereof of the present invention is reacted with cells or a homogenized solution thereof, a tissue or a homogenized solution thereof, cell culture supernatant, serum, or the like and then added with a carrier having the ability of specific binding to immunoglobulin, such as Protein G-Sepharose to precipitate antigen-antibody complex.

The sandwich ELISA method is a method in which, using the monoclonal antibody or the antibody fragments thereof of the present invention, of two kinds of monoclonal antibodies having different antigen-recognizing sites, one of the monoclonal antibodies or the antibody fragment thereof is adsorbed on a plate and the other of the monoclonal antibodies or the antibody fragment thereof is labeled with a fluorescent substance such as FITC or an enzyme such as peroxidase or biotin in advance. The antibody-adsorbing plate is reacted with cells or a homogenized solution thereof, a tissue or homogenized solution thereof, cell culture supernatant, serum, or the like and then reacted with the labeled monoclonal antibody or antibody fragment thereof to allow a reaction depending on the labeling substance.

The method of inhibiting the action of IL13 against the IL13 receptor-expressing cells by using the antibody or fragment thereof of the present invention may include at least one of the steps of: bringing the antibody or fragment thereof of the present invention into contact with the IL13 receptor-expressing cells; bringing IL13 into contact with the IL13 receptor-expressing cells; and detecting the inhibition of the action of IL13 against the IL13 receptor-expressing cells by the antibody or fragment thereof of the present invention. The step of detecting the inhibition of the action of IL13 against the IL13 receptor-expressing cells by the antibody or fragment thereof of the present invention may use an in vivo assay system in addition to the in vitro assay system described above. The in vivo assay system used herein means an evaluation system in which the antibody or fragment thereof of the present invention is administrated to the living body and the influence of the antibody or the antibody fragment on the function or status of the living body is detected. For instance, there is a system in which the antibody or fragment thereof of the present invention is administrated to a pathologic model animal and the influence on a severity index of pathologic condition is evaluated.

The present invention provides a reagent or kit for detecting or measuring IL13 receptor α1 in a test sample, the reagent or kit being characterized by containing the antibody or fragment thereof of the present invention. The reagent or kit can be based on the configuration of the detecting method described above.

### Examples

Hereinafter, the present invention will be described more specifically with reference to examples. However, these examples are provided by way of example, so that the present invention is not limited to any given example. In addition, abbreviations used in the following description are based on the conventional abbreviations accepted in the art.

### (Example 1) Preparation of Anti-Human Soluble IL13 Receptor α1 Antigen

### [Preparation of Administrating Antigens]

Expressions of Soluble IL13 Receptor α1-Fc and IL13 Receptor α1-His

A fusion protein (IL13 Rα1-Fc) between an extracellular domain of human IL13 receptor α1 (hereinafter, which may be abbreviated as IL13 Rα1) and an Fc fragment of human IgG and a protein (IL13 Rα1-His) attached with a hexahistidine tag on the C-terminal of an extracellular domain of IL13 Rα1 were prepared to be used as an administrating antigen and a screening antigen for antibody preparation. Furthermore, unless otherwise instructed, DNA manipulation was performed according to Molecular Coning, A Laboratory Manual 2nd ed., Maniatis T., et al., Cold Spring Harbor Laboratory Press (1989).

IL13 Rα1-Fc expression plasmid was prepared through genetic engineering by the following procedures. At first, on the basis of information about the sequence Y10659 of a human IL13 Rα1 gene registered in the DNA data bank Embl, sense primer 1 (containing a restriction enzyme EcoRI recognition sequence on the 5'-end side) and antisense primer 1 (containing a restriction enzyme HindIII recognition sequence on the 5'-end side) were synthesized and subjected to PCR using a human spleen cDNA library (Clontech, Co., Ltd.) as a template, obtaining a PCR product containing cDNA encoding an IL13 Rα1 extracellular domain --343 amino acids. A DNA fragment (EcoRI-HindIII fragment) obtained by digesting the PCR product with restriction enzymes EcoRI and HindIII was subcloned in pUC119 (Takara Shuzo Co., Ltd.). On the other hand, PCR was performed using plasmid pM1304 (described in WO97/42319) containing human IgG1Fc domain cDNA as a template, and thus human IgG1Fc domain cDNA capable of linking with the IL13 Rα1 extracellular domain in an inframe manner was obtained again. The sense primer 2 used for the PCR was one designed so as to contain a cDNA sequence encoding the N-terminal side of human IgG1Fc domain and a restriction enzyme HindIII recognition sequence on the 5'-end side. Also, the antisense primer 2 used was one deigned such that a restriction enzyme KpnI recognition sequence was arranged on the C-terminal side of the human IgGIFc domain. A DNA fragment (HindIII-KpnI) obtained by digesting the PCR product with the restriction enzymes HindIII and KpnI was subcloned in pUC119. Subsequently, the EcoRI-HindIII fragment and HindIII-KpnI fragment were cut out of the respective plasmids and then ligated together, followed by being introduced by replacing with an EcoRI-KpnI insert of a mammalian cell expression vector PEFN to prepare an expression vector plasmid pM1701 in which IL13 Rα1-Fc gene was ligated into the downstream of EF promoter. The inventors of the present invention have deposited the plasmid pM1701 to the International Patent Organism Depositary, National Institute of Advanced Industrial Science and Technology, Tsukuba Central 6, 1-1 Higashi 1-chome, Tsukuba-shi, Ibaraki-ken, Japan (Accession number FERM P-18632) on November 26, 2001, and transferred to the international depository authority according to the Budapest Treaty on November 18, 2002 (Accession number FERM BP-8238).

Furthermore, using the sense primer 1 described above and a primer (containing a restriction enzyme KpnI recognition sequence on the 5'-end side) containing the antisense sequence of cDNA encoding the IL13 Rα1 extracellular domain attached with hexahistidine on the C-terminal thereof, PCR was performed with IL13 Rα1-Fc expression plasmid as a template, and the PCR resulted in a product containing protein cDNA attached with a histidine tag on the C-terminal of IL13 Rα1 extracellular domain. The fragment digested with restriction enzymes EcoRI-KpnI in the PCR was replaced with the EcoRI-KpnI insert of the IL13 Rα1-Fc expression plasmid, and the resultant was provided as IL13 Rα1-His expression plasmid.

The resulting expression plasmid was introduced into COS cells by the method described below. That is, 50 µl of FuGENE6 (Roche Diagnostics, Co., Ltd.) and 12.5 µg of each plasmid DNA described above were mixed together according to the attached protocols, and the mixture was added to the COS cells grown in a semi-confluent manner in a 150-cm² flask. In the presence of 5% CO₂, the mixture was incubated for 3 days at 37°C, followed by collecting the culture supernatant. IL13 Rα1-His was purified from the culture supernatant using a nickel column (Hitrap-chelate, Amersham Pharmacia) and IL13 Rα1-Fc was purified using Protein-A column (Prosep-A, Millipore). The protein concentration was calculated using BSA as a standard product by a protein assay technology of Biorad Co., Ltd.

### [Preparation of Anti-IL13 Rα1 Antibody]

A mixture of 100 µg of purified soluble IL13 Rα1-Fc and Freund's complete adjuvant (Difco) at a proportion of 1 : 1 was administrated to the foodpads of 8-week-old female Wistar rats (purchased from SLC) and then the iliac lymph nodes were extracted from them after 3 weeks and lymphocytes were aseptically collected.

The resulting lymphocytes were mixed with mouse myeloma cells SP2/O-Ag14 (ATCC CRL1581) at a proportion of 5 : 1, followed by subjecting the mixture to cell fusion by means of polyethylene glycol 1500 (Sigma). After the fusion, the cells were suspended in 10% fetal serum / RPMI-1640 containing hypoxanthine, aminopterin, and thymidine and seeded in a 96-well plate (Nunc), followed by incubating under the conditions of 5% CO₂ and 37°C. When growing hybridomas were identified, the culture medium was replaced with an aminopterin-free medium.

One week after the cell fusion, the culture supernatant was sampled and hybridomas that produce antibodies to be bound to IL13 Rα1 were screed on a purified soluble IL13 Rα1-His-immobilizing plate. In other words, the purified soluble IL13 Rα1-His at a concentration of 1 µg/mL was immobilized on the plate (Maxisorp, Nunc) and blocked by PBS containing 0.1% BSA. Subsequently, the culture supernatant was added and reacted at 37°C for 1 hour, followed by washing with 0.9% physiological saline containing 0.05% Tween-20.

Peroxidase-labeled anti-rat immunoglobulin antibody (DAKO) was added to each well and reacted at 37°C for 1 hour. After washing, a tetramethylbenzidine coloring solution containing 0.02% hydrogen peroxide was added. Then, after a reaction for 10 minutes, the reaction was terminated by using 0.5 M sulfuric acid.

The absorbance was measured at a wavelength of 450 nm using a plate spectrophotometer (NJ-2100, Nippon Intermed). Wells each having an absorbance of 0.2 or more were selected as anti-IL13 Rα1 antibody-producing hybridomas.

The selected hybridomas were cloned by limiting dilution (Introduction to Monoclonal Antibody Laboratory Manual written by Tamiei Ando and Takeshi Chiba, Kodansha). 10 days after that, the screening was performed as described above to obtain 13 clones of anti-IL13 Rα1 antibody-producing hybridomas.

Subsequently, the hybridomas were incubated in RPMI-1640 containing 10% fetal bovine serum. After that, the cells were collected and the antibody production thereof was performed using Hybridoma-SFM (invitrogen), resulting in a culture supernatant containing monoclonal antibody. The cells were removed from the culture supernatant by means of paper filter and then purified through a protein-G column (Prosep-G, Millipore), resulting in 13 kinds of purified human IL13 Rα1 antibodies. Of the resulting purified antibodies, those having high reactivity were defined using a rat-typing kit available from ZYMED Co., Ltd. As a result, F997-20-1 antibody was IgG1·κ, F997-13-1 antibody was IgG1·κ, F997-10-1 antibody was IgG2b·κ, F994-7-2 antibody was IgG2a·κ, F997-18-3 antibody was IgG2b·κ, F997-17-1 antibody was IgG2a·κ, and F997-8-1 antibody was IgG2a·κ, respectively.

By the way, hybridomas F997-13-1 and F997-20-1 that produce F997-13-1 antibody and F997-20-1 antibody, respectively, were deposited to the International Patent Organism Depositary, National Institute of Advanced Industrial Science and Technology, Tsukuba Central 6, 1-1 Higashi 1-chome, Tsukuba-shi, Ibaraki-ken, Japan (Accession numbers FERM P-18633 and FERM P-18634, respectively) on November 26, 2001, and transferred to the international depository authority according to the Budapest Treaty on November 20, 2002 (Accession numbers FERM BP-8241 and FERM BP-8242, respectively). Hybridoma F997-10-1 that produces F997-10-1 antibody was internationally deposited to the same center on November 13, 2002 (Accession number FERM BP-8237).

### (Example 2) Measurement of Specificity and Affinity of Anti-IL13 Rα1 Antibody using BIACORE

### [Investigation on Specificity]

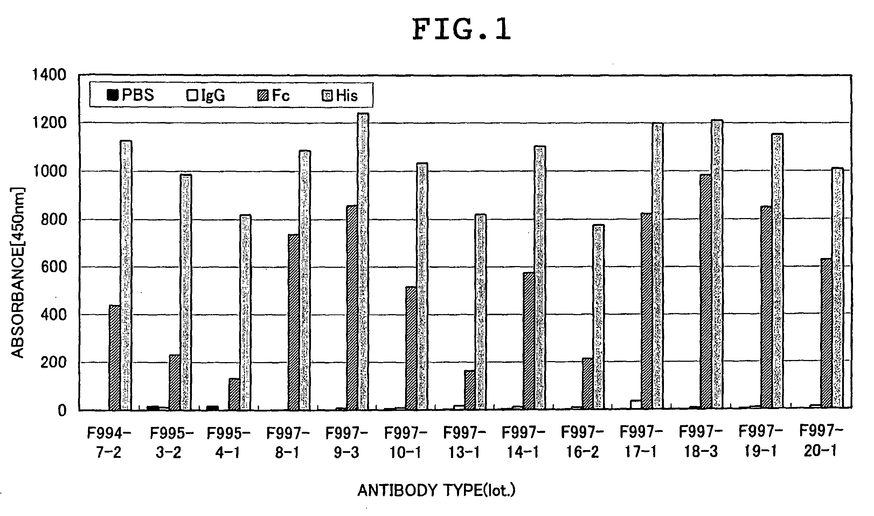

The human IL13 Rα1-Fc and human IL13 R-His prepared in Example 1, and human gamma-globulin (Cappel, Co., Ltd.) as a negative control were immobilized at 1 µg/ml in 96-well plates, respectively. After washing, blocking was performed by using a 0.1% BSA / phosphate buffer (pH 6.4). Subsequently, 1µg/ml of each antibody was diluted with a 0.1% BSA / phosphate buffer (pH 6.4) and then added to four places of no immobilized antigen (which may be abbreviated as PBS), human gamma globulin (which may be abbreviated as IgG), human IL13 Rα1-Fc (which may be abbreviated as Fc), and human IL13 Rα1-His (which may be abbreviated as His) and then reacted at 37°C for 1 hour. After washing with 0.9% NaCl containing 0.05% Tween-20, peroxidase-labeled anti-rat Igs antibody (DAKO) was added and then reacted in the same manner. Then, the plate was washed and then reacted for 10 minutes after the addition of a tetramethylbenzidine coloring solution containing 0.02% hydrogen peroxide, followed by terminating the reaction with 0.5 M sulfuric acid. The absorbance was measured at a wavelength of 450 nm using a plate spectrophotometer (NJ-2100, Nippon Intermed). The results were shown in Fig. 1. As is evident from Fig. 1, all of 13 kinds of the antibodies were specific to human IL13 Rα1-Fc and human IL13 Rα1-His, compared with PBS and IgG, and specifically bound to human L13 Rα1.

Subsequently, using BIACORE2000 (Biacore Co., Ltd.), the specificity to human IL13 Rα2 was studied. That is, at a flow rate of 20 µl/min, an NTA chip (Biacore Co., Ltd.) was treated with 5 µl of a 350-mM EDTA solution and then 5 µl of a 500-µM nickel solution was added. After that, 5 µl of 50-µg/ml Recombinant Human IL13 Rα2/Fc Chimera (R&D, Co., Ltd.) was added and bound. Furthermore, 5 µl of each antibody diluted to 50 µg/ml was added and the binding thereof was analyzed. Likewise, using Recombinant Mouse IL-13 Rα1/Fc Chimera (R&D, Co., Ltd.), cross-reactivity to mouse IL13 Rα1 was studied. As a result, out of the antibodies being studied, F997-20-1 antibody showed its cross-reactivity to human IL13 Rα2. In addition, the cross-reactivity to mouse IL13 Rα1 was identified in F997-20-1 antibody, F997-13-1 antibody, and F997-10-1 antibody. Here, RU is the unit representing a response used in a BIACORE device, so that 1,000 RU can represent that approximately 1.2 ng of a substance is bound.

**Table 1**

| Designation of antibody | Human IL13 Rα2 binding (RU) | Mouse IL13 Rα1 binding (RU) |
|---|---|---|
| F997-10-1 | 54 | 153 |
| F997-13-1 | 48 | 191 |
| F997-16-2 | 24 | 18 |
| F997-18-3 | 16 | 99 |
| F994-7-2 | 42 | 37 |
| F995-3-2 | 18 | 55 |
| F995-4-1 | 23 | 27 |
| F997-8-1 | 29 | 44 |
| F997-17-1 | 28 | 64 |
| F997-19-1 | 28 | 77 |
| F997-20-1 | 120 | 145 |

### [Investigation on Affinity]

The association constant of the anti-IL13 Rα1 antibody prepared was determined using BIACORE. That is, a CM5 chip (Biacore CO., Ltd.) was activated for 7 minutes according to the manual of Biacore Co., Ltd. and then 100 µg/ml of the human IL13 Rα1-Fc prepared in Example 1 was immobilized thereon. An HBS-EP buffer solution (Biacore Co., Ltd) was flown at a flow rate of 20 µl/min, and then the association constants of four kinds of the antibodies diluted to 2, 10, and 50 µg/ml with the same buffer solution were obtained. The binding antibody was dissociated with a 0.1-M glycine hydrochloric acid buffer solution (pH 3.0) and the chip was then used again. The resulting data was analyzed using analyzing software, BIA evaluation, Biacore Co., Ltd., and the dissociation constant (Kd) thereof was calculated. Consequently, the dissociation constants of four kinds of the investigated F997-10-1 antibody, F994-7-2 antibody, F997-13-1 antibody, and F997-20-1 antibody were listed in Table 2, and each of them showed sufficiently strong affinity to the human IL13 Rα1.

**Table 2**

| Designation of Antibody | Dissociation constant (M) |
|---|---|
| F997-10-1 | 1.65×10⁻⁹ |
| F994-7-2 | 1.5×10⁻¹⁰ |
| F997-13-1 | 4.1×10⁻⁹ |
| F997-20-1 | 1.07×10⁻⁹ |

### (Example 3) IL13 Binding Inhibition with Anti-IL13 Rα1 Antibody

For clarifying whether the antibody prepared could inhibit the binding of IL13 to the IL13 receptor α1, the analysis on binding was performed using BIACORE. That is, an NTA chip (Biacore CO., Ltd.) was treated with 5µl of a 350-mM EDTA solution at a flow rate of 20µl/min, followed by the addition of 5µl of a 500-µM nickel solution. After that, 5 µl of a 50-µg/ml Recombinant Human IL-13 Rα1/Fc Chimera (R&D, CO., Ltd.) was added and bound. Subsequently, 5µl of 50-µg/ml anti-IL13 Rα1 antibody was added and bound, and 5µl of 10-µg/ml-diluted human IL13 (R&D, Co., Ltd.) was added and the binding thereof was analyzed. In addition, using Recombinant Mouse IL-13 Rα1 / Fc Chimera (R&D, Co., Ltd.) and mouse IL13 (R&D, Co., Ltd.), the binding was analyzed similarly using an antibody showing the binding with mouse IL13 Rα1 in Example 2. Consequently, as shown in Table 3, of seven kinds of antibodies studied for the activity of inhibiting the biding of human IL13, four kinds of antibodies inhibited the binding between human IL13 and human IL13 receptor α1. On the other hand, of six kinds of antibodies studied for the activity of inhibiting the binding of mouse IL13 to mouse IL13 receptor α1, F997-13-1, F997-10-1, and F997-20-1 antibodies inhibited the binding between mouse IL13 and mouse IL13 receptor α1. The "%" in Table 3 represents a measured value such that the value is defined as "0%" when the IL13 and IL13 receptor α1 are bound at the time of adding control antibody and also defined as "100%" when the binding is completely inhibited. By the way, the value of the control antibody is a measured value when the rat IgG was added.

**Table 3**

| Designation of antibody | Human IL13/IL13 R binding inhibition rate (%) | Mouse IL13/IL13 R binding inhibition rate (%) |
|---|---|---|
| F997-13-1 | 28 | 14 |
| F997-10-1 | 62 | 10 |
| F997-20-1 | 44 | 30 |
| F997-18-3 | 6 | 4 |
| F997-19-1 | 6 | 3 |
| F994-17-1 | 6 | 5 |
| F994-7-2 | 22 | - |
| Control antibody | 0 | 0 |

### (Example 4) Measurement of Neutralization Activity by In Vitro Assay

TF-1 cells were typically incubated with 10% FBS, RPMI-1640, and GM-CSF (10 ng/ml) and the cell density was kept within the range of 10⁵ to 10⁶ /ml. The cells were incubated in a GM-CSF-free medium for one day and then subjected to an experiment.

The anti-IL-13 receptor α1 antibody obtained in Example 1 with 10% FBS and RPMI-1640 was prepared at twice as high a concentration as the final concentration (0.3 to 10 µg/ml) and then dispensed into a 96-well plate at 100 µl/well, followed by seeding the TF-1 cells at 1 x 10⁵ /100 µl and 2 x 10⁴ /100 µl. In the case of measuring the neutralization activity to GM-CSF and IL-4, the final concentration of the antibody was defined as 10 µg/ml. Then, the incubation was performed in a CO₂ incubator at 37°C for 3 hours, and IL-13 (2-2000 ng/ml) or IL-4 (2-2000 ng/ml) prepared at 20-fold concentration were added at a concentration of 10 µl/well in the plate seeded every 1 x 10⁵ /100 µl. In the plate seeded with 2 x 10⁴ /100 µl, GM-CSF (0.2 to 200 ng/ml) prepared at 20-fold concentration was added at a concentration of 10 µl/well. After the incubation had been performed for 48 hours, MTT (3-(4,5-dimethylthiazol-2-yl)-2,5-diphenyltetrazolium bromide; Dojindo Laboratories) (5 mg/mL in PBS) was added at a concentration of 25 µl/well. After the incubation had been performed for 3 hours, the culture supernatant was removed and formazan was then dissolved in a dissolving solution (100 µl/well), followed by measurement at OD570 nm. The dissolving solution was prepared by: dissolving 50 g of SDS (sodium dodecyl sulfate) in 400 mL pure water; adjusting the pH of the solution to 4.7 with 0.1 N HCl; and mixing the solution with 400 ml of DMF (dimethylformamide). Three examples were performed every group.

The neutralization activity of each antibody was represented in Table 4 as an antibody concentration required for shifting the dose-response curve of IL13 for the cell response by IL13 toward twice higher concentrations. The antibody concentration required for shifting the dose-response curve of IL13 for the cell response by IL13 toward twice higher concentrations can be expressed as 10^{-pA2} (g/mL) from the x value = pA2 when a regression line becomes y = 0. The regression line is obtained by calculating ED50 of the IL13 for each antibody concentration and plotting the antibody concentration expressed as -log (Ab)(Ab = g/ml) on the X axis and log [(A)/(A₀) - 1] (A = ED50 in the presence of antibodies, and A₀ = ED50 in the absence of antibody) on the Y axis, respectively.

**Table 4**

| Designation of antibody | Neutralization activity (µg/mL) |
|---|---|
| F995-3-2 | 2.6 |
| F997-18-3 | 1.5 |
| F997-13-1 | 1.3 |
| F997-20-1 | 1.0 |
| F997-8-1 | 0.8 |
| F997-17-1 | 0.8 |
| F997-19-1 | 0.8 |
| F994-7-2 | 0.4 |

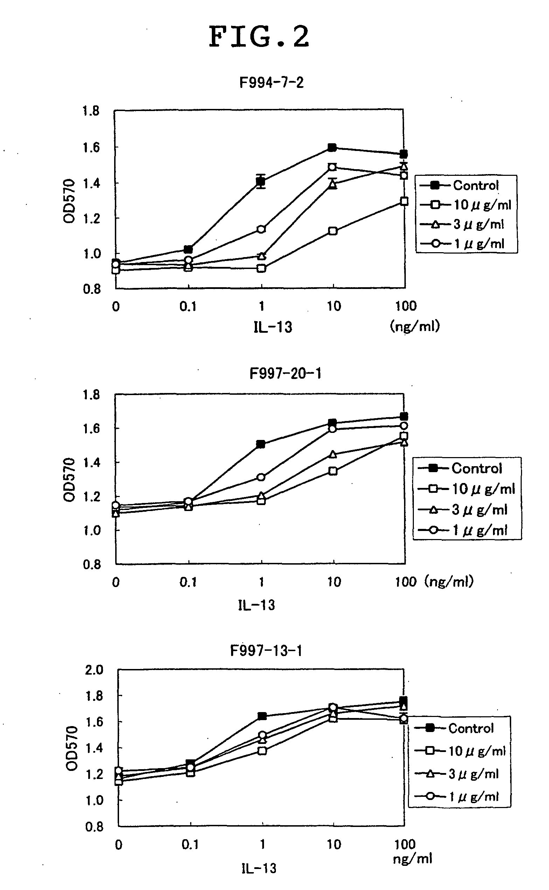

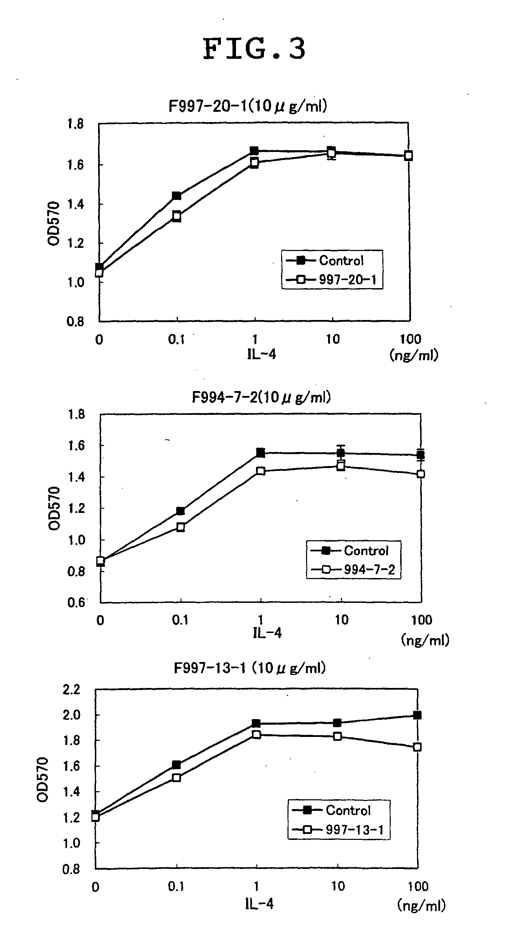

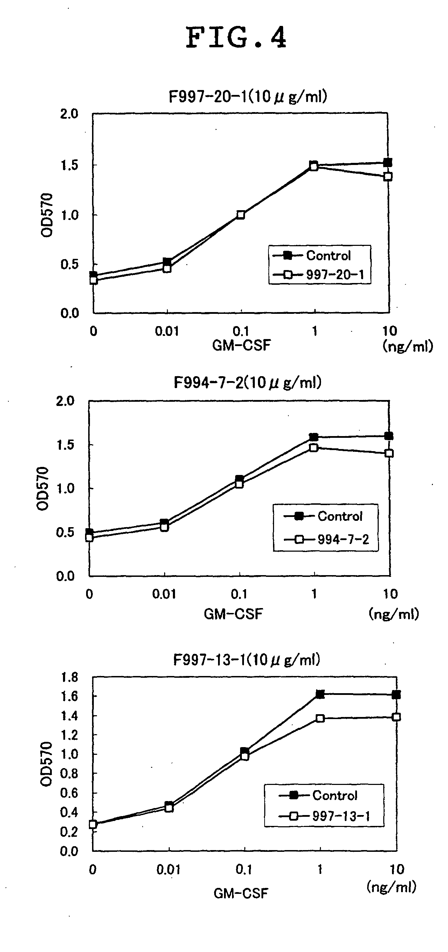

For F997-13-1, F997-20-1, and F994-7-2, the specific experimental results of the IL13-added group are shown in Fig. 2, and the specific experimental results of the IL4 or GM-CSF addition group are shown in Fig. 3 and Fig. 4. In the figures, the control represents an antibody-free group.

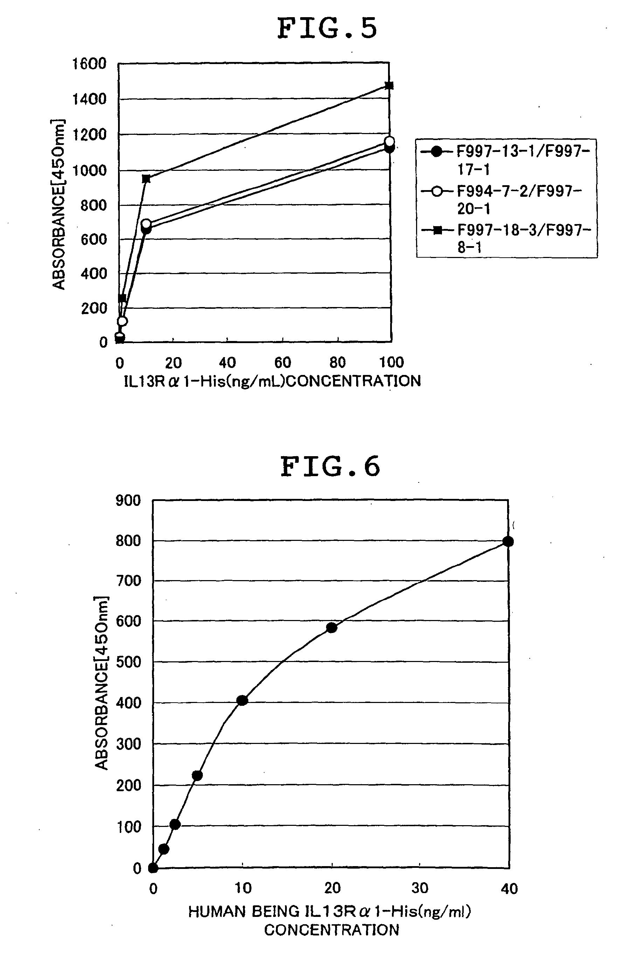

Fig. 5 summarizes the results of the inhibition activity to the cell response with IL13 and the inhibition activity to the cell response with IL4 and GM-CSF with respect to F997-13-1, F997-20-1, and F994-7-2.

**Table 5**

| | F997-13-1 | F997-20-1 | F994-7-2 |
|---|---|---|---|
| 10^{-pA2} (µg/mL) for cell response with IL 13 | 1.3 | 1.0 | 0.4 |
| Inhibition rate (%) at 10-µg/mL antibody concentration for cell response with IL 13 (1 ng/mL) | 57 | 87 | 107 |
| IC50 (µg/mL) for cell response with IL 13 (1 ng/mL) | 6.6 | 1.2 | 0.7 |
| IC50 (µg/mL) for cell response with IL 4 (1 ng/mL) | >10 | >10 | >10 |
| IC50(µg/mL) for cell response with GM-CSF (1 ng/mL) | >10 | >10 | >10 |

As shown in Fig. 3, Fig. 4, and Table 5, when the same experiments were performed using IL4 or GM-CSF as proliferation stimuli, even the addition of these antibodies at 10 µg/ml showed nearly no proliferation inhibition. That is, the experiments have shown that those antibodies inhibit the cell response by IL13, but they do not inhibit the cell response by IL4.

### (Example 5) Preparation of Measurement System for Soluble IL13 Receptor

### [Preparation of Sandwich ELISA System]

### (1) Search of Combination-Allowable Antibodies

For searching the combinations capable of producing sandwich ELISA out of the prepared anti-IL13 Rα1 antibodies, each purified antibody prepared in Example 1 was diluted to 10 µg/mL with a phosphate buffer solution (PBS). 50 µL of the diluted product was added to each well of an immuno plate (Maxisorb, NUNC), and the plate was treated at 45°C for 30 minutes to immobilize the antibodies. After washing with ion-exchanged water, blocking was performed by the addition of 100-µl PBS containing 0.1% BSA to each well. Then, a blocking solution was discarded. Subsequently, added was 25 µL each of purified soluble IL13 Rα1-His diluted to 1, 10, and 100 ng/mL with 0.1% BSA/PBS. 0.1% BSA/PBS was used for a blank. Subsequently, each peroxidase-labeled anti-IL13 Rα1 antibody was diluted to 3 µg/mL with a phosphate buffer solution (pH 6.4) containing 10% rabbit serum, 1% rat serum, 0.1% Tween-20, and 0.9% NaCl, and then 25 µL of the diluted product was added to each well. After a reaction at 37°C for 2 hours, the plate was washed 5 times with 0.9% NaCl containing 0.05% Tween-20 and then a tetramethylbenzidine solution containing 0.02% hydrogen peroxide was added to each well. Then, a reaction was proceeded at room temperature for 10 minutes and then terminated with a 0.5-M sulphuric acid solution. The absorbance was measured at a wavelength of 450 nm using a plate spectrophotometer (NJ-2100, Nippon Informed). Consequently, as shown in Fig. 5, three combinations of F997-13-1 antibody solid phase / F997-17-1 labeled antibody, F994-7-2 antibody solid phase / F997-20-1 labeled antibody, and F997-18-3 antibody solid phase / F997-8-1 labeled antibody were obtained as the combinations of antibodies capable of producing the sandwich EIA system.

### (2) Establishment of Sandwich ELISA System

Using the F994-7-2 antibody solid phase / F997-20-1 labeled antibody out of the resulting combinations, the sandwich ELISA system was prepared as described below. Purified F997-7-2 antibody was diluted to 10 µg/mL with PBS (pH 6.4) and 50 µL of the diluted product was added to each well of an immuno plate (Maxisorb, NUNC). After a reaction at 45°C for 30 minutes, the plate was washed 5 times with ion-exchanged water and blocking was performed by adding 100 µL of PBS (pH 6.4) containing 0.1% BSA to each well. The purified human IL13 Rα1-His was diluted to 1.25, 2.5, 5, 10, 20, and 40 ng/ml with rabbit serum to prepare standard preparations. The rabbit serum was used as a blank. The blocking agent in the plate was discarded and then the prepared standard preparations and the blank of the rabbit serum were dispensed in an amount of 25 µl at a time. Subsequently, the peroxidase-labeled F997-20-1 antibody was diluted to 4 µg/ml with a phosphate buffer solution (pH 6.4) containing 10% rabbit serum, 1% rat serum, 0.1% Tween-20, and 0.9% NaCl, and then 25µl of the diluted product was dispensed, followed by allowing a reaction at 37°C for 2 hours. Next, the plate was washed 5 times with physiological saline containing 0.05% Tween-20, followed by the addition of a tetramethylbenzidine solution containing 0.02% hydrogen peroxide to each well. After a reaction at room temperatures for 10 minutes, the reaction was terminated by using 0.5-M sulphuric acid solution. The absorbance at 450 nm was determined using a plate spectrophotometer (NJ-2100, Nippon Intermed) and a standard curve was prepared. The prepared standard curve was shown in Fig. 6. As is evident from the standard curve, a simplified measuring system having high sensitivity has been realized.

### (Example 6) Measurement of Soluble IL13 Receptor α1 in Blood

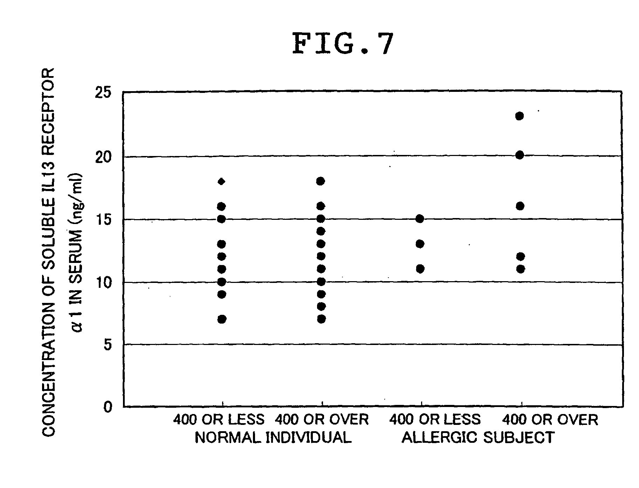

Serum measurement was performed using the measuring system described in Example 5 with respect to 32 cases of the normal individuals (male: 24 cases and female: 8 cases) and 8 cases of allergic subjects. In addition, the serum IgE concentration was measured by means of the sandwich ELISA system using anti-human IgE antibody (F271-15 antibody and peroxidase-labeled F271-15 antibody, Mochida Pharmaceutical Co., Ltd.). The measurements are shown in Fig. 7. In addition, the normal individuals and allergic subjects were classified into groups with a cut off value equal to a serum IgE concentration of 400 U/mL. The average value +/- SD of each group was shown in Table 6. The soluble IL13 receptor α1 concentration in the normal individuals at an IgE concentration of 400 U/mL or less (in Fig. 7, abbreviated as 400 or less) was 12.8 +/- 3.2 ng/ml, so that there is no difference compared with a soluble IL3 receptor α1 concentration of 11.9 +/- 2.9 ng/ml in the normal individuals at an IgE concentration of 400 U/mL or over. In the allergic subjects, the soluble IL13 receptor α1 concentration at an IgE concentration of 400 U/mL or less was 13.0 +/- 1.6 ng/ml, so that there is no difference compared with the concentration of the normal individuals. In the allergic subjects, the soluble IL13 receptor α1 concentration at an IgE concentration of 400 U/mL or over was 16.4 +/- 4.6 ng/ml: the concentration tends to be higher than those of other groups. Therefore, an allergic subject with an increasing serum IgE concentration was also increased in soluble IL13 receptor α1 concentration. The results described above show that, using the prepared measuring system, the measurement of soluble IL13 receptor α1 concentration in serum is useful for the understanding of the disease state of an allergic subject and for the choice of therapy.

**Table 6**

| Classification | IgE concentration of 400 U/mL or less | IgE concentration of 400 U/mL or over |
|---|---|---|
| Normal individual | 12.8+/-3.2ng/ml | 11.9+/-2.9ng/ml |
| Allergic subject | 13.0+/-1.6ng/ml | 16.4+/-4.6ng/ml |

### (Example 7) Inhibition of Phosphorylation of STAT6 with Anti-IL13 Receptor α1 Antibody

The inventors of the present invention have considered whether two kinds of antibodies (F997-10-1 and F997-13-1) prepared in Example 1 bind with IL13 receptors of mouse spleen monocytes and inhibit the phosphorylation of STAT6 under the stimulus of IL13. At first, the spleen was removed from a Balb/c mouse (male, 8 weeks old, SLC) and centrifuged at 1,000 rpm for 10 minutes. Then, the spleen was resuspended in a Tris-NH₄ solution for removing erythrocytes from monocytes (Shunsuke Migita, et al., Ed. Experimental Operation Methods of Immunology, Nankodo Co., Ltd., p560) to hemolyze the erythrocytes. After having been recentrifuged at 1,000 rpm for 10 minutes, the spleen was washed twice with RPMI1640 (SIGMA) and resuspended in an RPMI1640 medium containing 10% fetal bovine serum, followed by measuring the cell concentration. The cell concentration was adjusted to 1 x 10⁶ cells/ml and 1 ml of the resultant solution was dispensed into each well of a 24-well plate. Subsequently, two kinds of anti-IL13 R antibodies were added to final concentrations of 0, 10, 30, and 100 µg/ml. In addition, the rat IgG was added as a negative control to final concentration of 10 µg/ml. After the resultant had been allowed to stand at room temperature for 30 minutes, a solution containing mouse spleen monocytes was collected and centrifuged at 5,000 rpm for 5 minutes, followed by removing the supernatant. On ice, an SDS buffer solution (5 mM NaVO₃, 10mM NaF/Tris-SDS-β-mercaptoethanol) containing 50 µl of a phosphatase inhibitor was added to the precipitate to dissolve the cells therein. Subsequently, the solution was subjected to ultrasonic treatment at 50 Hz for 15 seconds and then to thermal denaturation at 99°C for 5 minutes. Furthermore, the resultant was centrifuged at 15,000 rpm for 5 minutes and then 20 µl of the supernatant thereof was used in SDS-PAGE / Western blotting. The supernatant was added to an equal volume of a reduction electrophoretic buffer (OWL) and the mixture was applied to e-PAGEL (5-20%, ATTO), and electrophoresed at 40 mA/gel for 50 minutes at room temperature. After the electrophoresis, protein was transferred at 150 mA/gel to a PVDF membrane (Millipore) at 4°C according to the manual from Millipore and was then subjected to blocking at room temperature for 60 minutes using a 5% skim milk / 0.1% Tween-20 / 50 mM Tris-HCl / 0.9% NaCl (pH 7.4) solution (hereinafter, referred to as TBS). A 667-fold dilution of anti-phosphorylated STAT6 antibody (Daiichi Kagaku, 9361S) with an antibody-diluting solution (5% BSA / 0.1% Tween-20 / 50 mM TBS) was added to the membrane and reacted for overnight at 4°C (first order reaction). Next day, the membrane was washed three times with 0.1% Tween-20 / 50 mM TBS and then a 2,000-fold dilution of peroxidase-labeled anti-rabbit immunoglobulin antibody (DAKO, P 0448) with an antibody-diluting solution (5% BSA / 0.1% Tween-20 / 50 mM TBS) was added to the washed membrane and reacted for 30 minutes at room temperature (second order reaction). The membrane was washed three times with 0.1% Tween / 50 mM TBS and allowed to emit light by ECL kit (Amersham Biosciences) and to expose HyperFilm (Amersham Biosciences), followed by developing. Consequently, as shown in Table 7, the addition of F997-10-1 antibody or F997-13-1 antibody at a concentration of 30 or 100 µg/ml reduces the strength of phosphorylated STAT6, compared with the addition of negative control antibody. Thus, the F997-10-1 antibody or F997-13-1 antibody is connected to the IL13 receptor, showing that it inhibits the phosphorylation of STAT6 by the stimulation of IL13.

**Table 7**

| | N.C | F997-10-1 | | F997-13-1 | |
|---|---|---|---|---|---|
| | | 30 µg/ml | 100 µg/ml | 30 µg/ml | 100 µg/ml |
| Band strength of phosphorylated STAT6 | +++ | ++ | + | +++ | ++ |

### (Example 8) Determination of CDR Sequence of Anti-IL13 Receptor α1 Antibody Variable Region

The complementarity determining regions (hereinafter, referred to as CDR) of three kinds of anti-IL13 receptor α1 antibodies prepared in Example 1 were determined. In other words, F997-10-1 antibody-producing hybridoma, F997-13-1 antibody-producing hybridoma, and F997-20-1 antibody-producing hybridoma were incubated according to Example 1. When the cell concentration reached 2 x 10⁵ cells/ml, 50 ml of each culture medium was collected and washed with Dulbecco's phosphate buffered saline (pH 7.4, Sigma) (hereinafter, referred to as PBS-). Then, mRNA was extracted from the collected cells using TRIzol (Invitrogen). Next, according to the manual of Superscript First-strand synthesis System (Invitrogen), cDNA was synthesized from mRNA using oligo dT (Invitrogen) and antisense primers (for H chain: HAS-1 (SEQ ID NO: 18) and for L chain: KAS-1 (SEQ ID NO: 19)). The resulting cDNA was used as a template and then PCR was conducted using Heavy Primer / Heavy Primer 2 (Amersham Biosciences) as the H chain and Light Primer Mix (Amersham Biosciences) as the L chain. PCR was performed using platinum Taq polymerase (Invitrogen) under the conditions of 30 cycles of 94°C - 30 seconds, 55°C - 30 seconds, and 72°C - 1 minute by means of PTC-200 Peletier Thermal Cycler (MJ Research). The bands of the amplified DNA were identified by means of 2% agarose, and the PCR product was purified through a spin column (Sigma). The purified PCR product and pT7-Blue T vector (Novagen) were mixed together and the mixture was subjected to a ligation reaction at 16°C for 30 minutes using a Ligation Kit ver. II (TAKARA). Using the reaction solution, transformation was performed on competent cell E.coli (JM109, TAKARA) and then the transformed product was seeded on an LB plate containing X-Gal, IPTG, or the like and left standing overnight. White colonies being generated were picked up. Then, the use of Ex Taq Polymerase (TAKARA), U-19 mer primer (SEQ ID NO: 21), and T7 promoter primer (SEQ ID NO: 20, Novagen) confirmed that the insert was introduced in the vector by colony direct PCR. Subsequently, the insert-identified colony was incubated overnight in an LB culture medium and then plasmid was purified using QIAGEN plasmid mini kit (QIAGEN). The purified plasmid was reacted with DYEnamic ET terminator cycle sequencing kit (Amersham Biosciences) using U-19 mer primer and T7 promoter primer. After the reaction, the reaction product was analyzed using the sequencer ABI PRISM 3100 (Applied Biosystems).

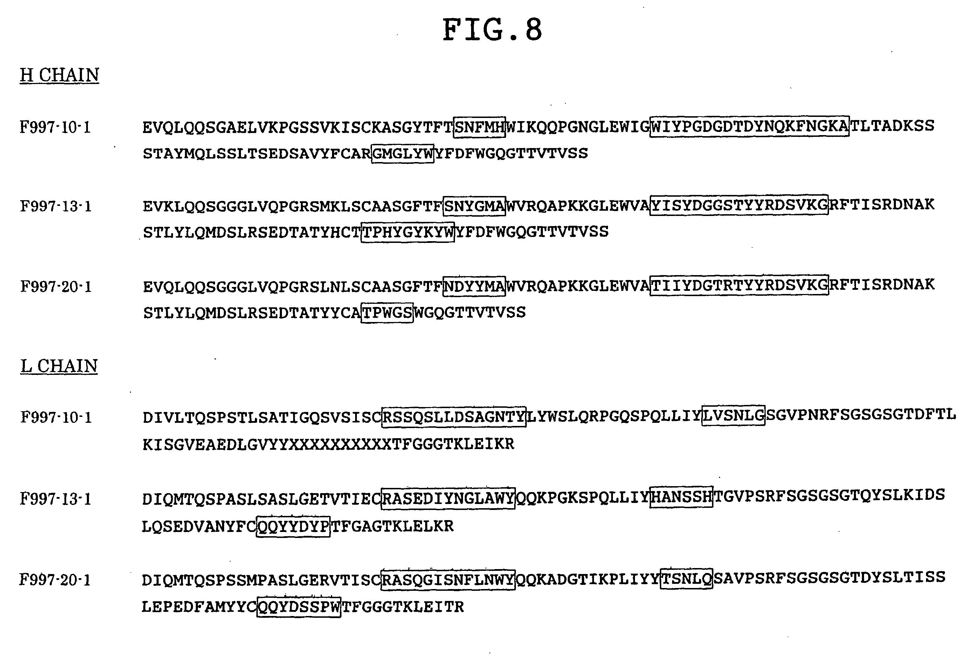

Heavy chain and light chain variable region gene sequences of F997-10-1 antibody, F997-13-1 antibody, and F997-20-1 antibody were represented by SEQ ID NO: 22 (F997-10-1 heavy chain), SEQ ID NO: 23 (F997-10-1 light chain), SEQ ID NO: 24 (F997-13-1 heavy chain), SEQ ID NO: 25 (F997-13-1 light chain), SEQ ID NO: 26 (F997-20-1 heavy chain), and SEQ ID NO: 27 (F997-20-1 light chain). Furthermore, the translated amino acid sequences were represented in Fig. 8 and by SEQ ID NO: 28 (F997-10-1 heavy chain), SEQ ID NO: 29 (F997-10-1 light chain), SEQ ID NO: 30 (F997-13-1 heavy chain), SEQ ID NO: 31 (F997-13-1 light chain), SEQ ID NO: 32 (F997-20-1 heavy chain), and SEQ ID NO: 33 (F997-20-1 light chain), respectively. The determined CDR sequences were shown in Table 8. The base sequence of the third CDR of the F997-10-1 light chain variable region is not yet revealed.

**Table 8**

| Designation of antibody | Chain | CDR | Amino acid sequence | SEQ ID NO |
|---|---|---|---|---|
| F997-10-1 | H chain | CDR 1 | SNFMH | 1 |
| | | CDR 2 | WIYPGDGDTDYNQKFNGKA | 2 |
| | | CDR 3 | GMGLYW | 3 |
| | L chain | CDR 1 | RSSQSLLDSAGNTY | 4 |
| | | CDR 2 | LVSNLG | 5 |
| | | CDR 3 | - | - |
| F997-13-1 | H chain | CDR 1 | SNYGMA | 6 |
| | | CDR 2 | YISYDGGSTYYRDSVKG | 7 |
| | | CDR 3 | TPHYGYKYW | 8 |
| | L chain | CDR 1 | RASEDIYNGLAWY | 9 |
| | | CDR 2 | HANSSH | 10 |
| | | CDR 3 | QQYYDYP | 11 |
| F997-20-1 | H chain | CDR 1 | NDYYMA | 12 |
| | | CDR 2 | TIIYDGTRTYYRDSVKG | 13 |
| | | CDR 3 | TPWGS | 14 |
| | L chain | CDR 1 | RASQGISNFLNWY | 15 |
| | | CDR 2 | TSNLQ | 16 |
| | | CDR 3 | QQYDSSPW | 17 |

### (Example 9) Preparation of Human Chimeric Antibody

The V region having the antigen-binding activity was originated from F997-10-1 antibody, F997-13-1 antibody, or F997-20-1 antibody, i.e., originated from rat antibody. Thus, an antibody with less antigenecity to a human being can be obtained by preparing the C region as an antibody originated from a human being (chimeric antibody). The donors for the V region are not limited to the antibodies described above but may include any of antibodies as far as they are the anti-IL13 receptor α1 antibodies of the present invention having the activity of inhibiting the cell response by IL13. Many chimeric antibodies have been developed since the report of Morrison et al., 1984 (Proc, Natl. Acad. Sci. USA, 81: 6851, 1984).

### (1) Cloning of Antibody Gene

Hybridoma F997-10-1, hybridoma F997-13-1, or hybridoma F997-20-1 is incubated and cells are prepared therefrom. The obtained cells are washed with PBS- (Sigma) and then the total RNA is isolated and purified using Isogene (Nippon Gene Co., Ltd.). Next, cDNA is synthesized using Oligo-dT primer and SuperScript II System (Invitrogen). A sense primer is synthesized on the basis of amino acid sequences of the amino terminals of the heavy chain and light chain. Furthermore, the heavy chain antisense primer is prepared on the basis of the sequence of framework 4 and the light chain antisense primer is prepared on the basis of Vκ sequence. After PCR, the DNA fragment is incorporated into the TA cloning vector (Invitrogen) to analyze the sequence.

### (2) Construction of Rat-Human Heavy Chain and Light Chain Expression Vector

At first, a base sequence that encodes the N-terminal side of the CH1 region of human immunoglobulin G1 is synthesized as a sense primer, and the antisense primer synthesizes a region that includes the sequence of 3'-non translation region of the human immunoglobulin G1. The human immunoglobulin CH region is amplified by the PCR reaction from the Human Spleen 5'-Stretch cDNA Library (Clontech) using the sense primer and the antisense primer. In addition, the heavy chain sense primer is prepared such that a sequence that encodes the EcoRI sight is included in a base sequence that encodes the heavy chain region of the above (1) and an amino acid sequence that encodes the N-terminal side of the CH1 region of the human immunoglobulin G1. The antisense primer is prepared so as to contain: a base sequence that encodes an amino acid sequence located on the C-terminal side of the CH3 region of the human immunoglobulin G1; and the BamHI site. Those chimeric primers are combined together and then the human immunoglobulin CH region is incorporated such that the orientation of the region corresponds to the rat VH region. The resulting PCR product is digested with restriction enzymes and the DNA fragment is incorporated into the expression vector pcDNA 3.1 (Invitrogen) to prepare a rat-human heavy chain expression plasmid. An expression plasmid containing a chimeric antibody gene having the CL region of a human being and the light chain region originated from a rat is simultaneously constructed.

### (3) Preparation of Chimeric Antibody

For preparing a transformant, each of the expression plasmids is cut with restriction enzymes so as to become linear. Next, the gene is introduced into SP2/O-ag14 (ATCC CRL1581) using the gene pulser (BIORAD) or the like. The cells that produce the target antibody are sorted on the basis of the presence of a rat-human chimeric antibody to be produced in the supernatant after incubation. Specifically, about 20 µg of the linear DNA fragment is electroporated into 1 x 10⁷ cells at 360 V with a capacitance of 25 µFD. Subsequently, the cells are planted in a 96-well plate and incubated for two days. After that, D-MEM (Sigma) containing 10% FCS, 1 x HT (Invitrogen), 0.2 mg/ml G-418 is added to the cells for the selection of plasmid fragment-incorporating cells and then the cells are incubated for additional two weeks. When the cells are brought into a confluent state, they are incubated in a serum-free medium (Hybridoma-SFM, Invitrogen) and then the culture supernatant is purified through Protein A column (Prosep-A, Millipore), resulting in a purified chimeric antibody.

For the resulting chimeric antibody, the activity of binding to IL13 receptor α1 is confirmed by the method in Example 2, and also the neutralization activity of inhibiting the cell response by IL13 is confirmed by the method of Example 4.

### (Example 10) Preparation of Human CDR Grafting Antibody (1)

### (1) Computer Modeling of Antibody Variable Region

For retaining high affinity in humanized antibody, a framework residue is selected according to the general method of Queen et al. (Proc. Natl. Acad. Sci. USA 86: 10029, 1989). A sequence having high framework homology in rat F997-10-1 antibody, F997-13-1 antibody, or F997-20-1 antibody is selected as a human sequence on the basis of the kappa light chain and heavy chain sequence data base of kabat et al. (Sequences of proteins of immunological interest, 5th ed., U.S. Department of Health and Human Services, 1991). Furthermore, the most suitable modification of amino acids in the framework is performed on the basis of computer analysis. Specifically, the molecular model of a variable region in F997-10-1 antibody, F997-13-1 antibody, or F997-20-1 antibody is constructed using the computer program ENCAD (Levitt, J. Mol. Biol. 168, 595 (1983). In the human Eu antibody molecular model obtained from an antibody database (Stephens et al., Immunology 85 (4), 668-674 (1995)), a CDR sequence of F997-10-1 antibody, F997-13-1 antibody, or F997-20-1 antibody is transplanted in FR. Substitution to an amino acid originated from rat antibody is performed at a position in the computer model where the substitution of amino acids is expected to improve the contact between CDR and FR in an FR region where CDR and FR show significant contact with each other unlike the original human antibody model. In addition, any amino acid residue in FR which appears rarely at such a position in the human antibody database is substituted with a human consensus amino acid on such a position. The good or bad of amino acid substitution is confirmed by the actual activity, so that several kinds of antibodies having different types of amino acid substitution will be prepared.

### (2) Construction of Humanized Antibody

Constructed according to the sequence selected in (1) is a gene that encodes an amino acid sequence containing a signal peptide, splice-providing signal, and restriction site (e.g., XbaI). For the constructed gene, several kinds of synthetic nucleotides (almost 80 nucleotide length) are prepared so as to overlap. That is, oligo nucleotides are paired together, annealed, and elongated with a Klenow fragment of DNA polymerase, resulting in a double strand fragment. The fragment is denatured to obtain single strands. Then, likewise, the single strands are annealed and then elongated with a Klenow fragment of DNA polymerase to obtain a double strand fragment that encodes the total length of a gene. The obtained fragment is amplified by PCR with Taq polymerase and purified. Then, the fragment is purified by cutting the fragment with a restriction enzyme (e.g., XbaI). A part extending from CH1 exon to CH3 exon of a human γ1 gene of the purified fragment is inserted into the XbaI site of plasmid pVg1 (Co et al., J. Immunol. 148: 1149 (1992)) having an XbaI-BamHI fragment in a constant region gene. By the similar operation, it is possible to insert the fragment into a plasmid that contains a constant region gene of γ4. In addition, when the number of amino acids to be substituted is small, the fragment can be prepared by the introduction of site-specific mutation and introduced into an expression plasmid. A light chain variable region sequence can be constructed in the same way as described above. In this case, pVk vector used is one containing a human Cκ region.

For making an antigen-producing transformant, heavy chain and light chain plasmids are linearized by cutting the plasmids with restriction enzymes (BamHI and FspI in the case of pVk plasmid) and then introduced into mouse myeloma cells SP2/O-Ag14 (ATCC CRL1581) using a gene pulser (BIORAD). Specifically, about 20 µg of the linearized DNA fragment is electroporated into 1 x 10⁷ cells at 360 V with a capacitance of 25 µFD. Next, the cells are planted in a 96-well plate and incubated for two days. After that, for selecting the cells in which the plasmid fragments are incorporated, D-MEM (Sigma) containing 10% FCS, 1 x HT (Invitrogen), 0.25 mg/ml Xanthine, and 1 µg/ml Mycophenolic acid is added to the cells and the cells are incubated for additional two weeks. Selected from the antibodies appearing in the supernatant after the incubation is the desired humanized F997-10-1 antibody, humanized F997-13-1 antibody, or humanized F997-20-1 antibody-producing cell line. That is, the antibody bound to the immobilized IL13 receptor α1 is detected by peroxidase-labeled anti-human IgG1 or IgG4 antibody. The selected cell line is incubated in a 10%-FCS-containing culture medium until the cell line becomes confluent, followed by replacing with a serum-free culture medium (Hybridoma SFM, Invitrogen). The culture supernatant is collected and allowed to bind to Protein A (Prosep-A, Millipore), followed by being eluted out with 0.1 µM glycine HCl (pH 3.0). The purified antibody is dialyzed in PBS- (Sigma) and the antibody concentration is evaluated from an absorbance at 280 nm (1 mg/mL human antibody shows an absorbance of 1.3).

### (3) Evaluation of Humanized Antibody

For confirming that the humanized antibody has the same activity as that of the original rat antibody, the activity of binding to IL13 receptor α1 is confirmed by the method of Example 2 and then the neutralization activity thereof to inhibit cell response by IL13 is confirmed by the method of Example 4.

### (Example 11) Preparation of Human CDR Grafted Antibody (2)

For allowing a CDR sequence to be transplanted to retain a suitable domain structure having the activity in humanized antibody, the original FR region sequence is also transplanted. Finding an amino acid involved in retaining the CDR domain structure is performed by analyzing the properties (e.g., hydrophobicity, hydrophilicity, acidity, basicity, and molecular size) of amino acids in FR and by computer modeling. That is, the modeling is performed using the software QUANTA/CHARMm or Modeler (Molecular Simulations) to be activated on the Silicon Graphics. The three dimensional structures of the antibodies having high homology with the VH and VL regions of F997-10-1 antibody, F997-13-1 antibody, or F997-20-1 antibody are searched from the human antibody sequences registered in the Brookhaven Protein Data Bank (PDB), and then the three dimensional structure of F997-10-1 antibody, F997-13-1 antibody, or F997-20-1 antibody is estimated on the basis of the search results. On the estimated three dimensional structure, an amino acid group (a first group) in the FR region bound to CDR of the heavy and light chains through a hydrogen bond is selected, and an amino acid group (a second group) in the FR region further bound to them through a hydrogen bond is selected. Similarly, an amino acid group (a first group) in the FR region which may be bound to CDR through an energy bond such as electrostatic interaction or van der Waals force and an amino acid group (a second group) in the FR region which may be further bound to them are selected. The amino acid groups in the FR region thus selected are transplanted into a human antibody sequence together with the CDR amino acid. However, the transplantation of amino acids is canceled if there is any sequence which does not exist in amino acids of variable regions of the human antibody sequence obtained by the classification of Kabat et al. (Sequences of proteins of immunological interest, 5th ed., U. S. Department of Health and Human Services, 1991), NCBI (National Center for Biotechnology Information), or the like. According to the information thus gained, the sequences to be grafted into the VH and VL of the human antibody sequence are determined, so that a gene used for the preparation of a humanized antibody will be constructed.

The constructed gene is prepared by a method of combining the kit from Amersham (Oligonucleotide-directed in vitro mutagenesis system version 2) with the PCR method, a method for amplification by combining several kinds of synthesized long-chain nucleotides, or a method involving amplifying VH or VL gene of chimeric antibody using several kinds of primers as templates and then obtaining the total length of the gene fragment using the amplified gene fragment as a template. The resulting amplified gene fragment is introduced into the restriction enzyme site of the plasmid pVg1 described in Example 10 or plasmid pVk containing Vκ to prepare an expression plasmid. The prepared plasmid is introduced into cells by the method described in Example 10 and a transformant is obtained, and similarly a purified antibody is prepared. Furthermore, likewise, the antibody is evaluated.

### Industrial Applicability

An increase in expression of IL13 has been observed in local condition of the inflammatory disease. Therefore, the antibody of the present invention which inhibits the binding between IL13 and IL13 receptor and neutralizes the action of IL13 is a therapeutic agent effective to inflammatory diseases. IL4 and IL13 have the overlapping physiological activities. IL13 is mainly found in the local condition of disorder and involved in inflammatory reaction. On the other hand, IL4 may play a homeostatic role such as class switching of antibody production or Th2-differentiation of immunocytes. The antibody of the present invention, which selectively inhibits the cell response by IL13 but does not inhibit the cell response by IL4, is capable of preventing unexpected side effects involved in the inhibition against the action of IL4 in addition to the original purpose of inhibiting the local inflammatory reaction by inhibiting the action of IL13. The antibody of the present invention selectively inhibiting the action of IL13 can effectively prevent the local inflammatory reaction without causing any side effect.

## Claims

1. An anti-IL13 receptor α1 antibody, which has an activity of inhibiting a cell response by IL13.

2. An anti-IL13 receptor α1 antibody, **characterized in that** the anti-IL13 receptor α1 antibody has an activity of inhibiting a cell response by IL13 but does not inhibit a cell response by IL4.

3. The anti-IL13 receptor α1 antibody, according to Claim 1 or 2, wherein the anti-IL13 receptor α1 antibody is bound to an IL13 receptor α1 at a dissociation constant of at least 10 nM.

4. The anti-IL13 receptor α1 antibody, according to any one of Claims 1 to 3, wherein the cell response by IL13 is a cell proliferation of TF-1 cells, and the activity of inhibiting the cell response by IL13 is at least one activity selected from the group consisting of (a-1) to (a-3) below:
(a-1) an antibody concentration required for shifting a dose-response curve of IL13 in the cell response by IL13 toward a twice higher concentration is 10 µg/mL or less;
(a-2) an IC50 against the cell response in the presence of IL13 at a concentration of 1 ng/mL is 10 µg/mL or less; and
(a-3) an antibody concentration of 10 µg/mL inhibits 50% or more of the cell response in the presence of IL13 at a concentration of 1 ng/mL.

5. The anti-IL13 receptor α1 antibody, according to any one of Claims 1 to 4, wherein the anti-IL13 receptor α1 antibody is a monoclonal antibody.

6. The anti-IL13 receptor α1 antibody, according to any one of Claims 1 to 5, **characterized in that** the anti-IL13 receptor α1 antibody has a heavy chain variable region selected from the group consisting of (a) to (c) below:
(a) a heavy chain variable region **characterized by** having a first complementarity determining region consisting of an amino acid sequence described in SEQ ID NO: 1, a second complementarity determining region consisting of an amino acid sequence described in SEQ ID NO: 2, and a third complementarity determining region consisting of an amino acid sequence described in SEQ ID NO: 3;
(b) a heavy chain variable region **characterized by** having a first complementarity determining region consisting of an amino acid sequence described in SEQ ID NO: 6, a second complementarity determining region consisting of an amino acid sequence described in SEQ ID NO: 7, and a third complementarity determining region consisting of an amino acid sequence described in SEQ ID NO: 8; and
(c) a heavy chain variable region **characterized by** having a first complementarity determining region consisting of an amino acid sequence described in SEQ ID NO: 12, a second complementarity determining region consisting of an amino acid sequence described in SEQ ID NO: 13, and a third complementarity determining region consisting of an amino acid sequence described in SEQ ID NO: 14.

7. The anti-IL13 receptor α1 antibody, according to Claim 6, **characterized by** comprising a light chain variable region having: a first complementarity determining region consisting of one amino acid sequence selected from the group consisting of amino acid sequence of SEQ ID NO: 4, amino acid sequence of SEQ ID NO: 9, and amino acid sequence of SEQ ID NO: 15; a second complementarity determining region consisting of one amino acid sequence selected from the group consisting of amino acid sequence of SEQ ID NO: 5, amino acid sequence of SEQ ID NO: 10, and amino acid sequence of SEQ ID NO: 16; and a third complementarity determining region consisting of one amino acid sequence selected from the group consisting of amino acid sequence of SEQ ID NO: 11 and amino acid sequence of SEQ ID NO: 17.

8. An antibody, which is produced by hybridoma F997-20-1, hybridoma F997-13-1, or hybridoma F997-10-1.

9. The anti-IL13 receptor α1 antibody according to any one of Claims 1 to 7, wherein the anti-IL13 receptor α1 antibody is a humanized antibody.

10. The anti-IL13 receptor α1 antibody according to Claim 9, wherein the humanized antibody comprises a human chimeric antibody.

11. The antibody according to Claim 10, wherein the human chimeric antibody is a human chimeric antibody consisting of: the heavy chain variable region and the light chain variable region of the antibody according to any one of Claims 1 to 7; and a heavy chain constant region and a light chain constant region of a human antibody.

12. The antibody according to Claim 10, wherein amino acid sequences of the heavy chain variable region and the light chain variable region are the same as amino acid sequences of a heavy chain variable region and a light chain variable region of an antibody produced by hybridoma F997-20-1, hybridoma F997-13-1, or hybridoma F997-10-1.

13. The anti-IL13 receptor α1 antibody according to Claim 9, wherein the humanized antibody comprises a human complementarity determining region grafted antibody.

14. The antibody according to Claim 13, wherein the human complementarity determining region grafted antibody is a human complementarity determining region grafted antibody consisting of: the heavy chain complementarity determining region and the light chain complementarity determining region of the antibody according to any one of Claims 1 to 7; and a heavy chain constant region, a light chain constant region, and a framework region of a human antibody.

15. The antibody according to Claim 14, wherein amino acid sequences of the heavy chain complementarity determining region and the light chain complementarity determining region have the same amino acid sequences as amino acid sequences of a heavy chain complementarity determining region and a light chain complementarity determining region of an antibody produced by hybridoma F997-20-1, hybridoma F997-13-1, or hybridoma F997-10-1.

16. The anti-IL13 receptor α1 antibody according to any one of Claims 1 to 7, wherein the anti-IL13 receptor α1 antibody is a human antibody obtained from a human antibody phage library or a human antibody-producing transgenic animal.

17. An anti-IL13 receptor α1 antibody fragment, comprising a part of the antibody according to any one of Claims 1 to 8 with an activity of inhibiting a cell response by IL13.

18. A hybridoma, which produces the antibody according to any one of Claims 1 to 7.

19. A hybridoma F997-20-1, hybridoma F997-13-1, or hybridoma F997-10-1.

20. A method of detecting an IL13 receptor α1 in a test sample using the antibody or the antibody fragment according to any one of Claims 1 to 17.

21. A method of inhibiting an action of IL13 to an IL13 receptor-expressing cell using the antibody or the antibody fragment according to any one of Claims 1 to 17.


SEQUENCE LISTING

<110> Mochida Pharmaceutical Co., Ltd.

<120> Anti-IL13receptor alpha 1 neutralizing antidody

<130> PCT185

<160> 33

<170> PatentIn version 3.1

<210> 1

<211> 5

<212> PRT

<213> rat

<400> 1

Ser Asn Phe Met His

1               5

<210> 2

<211> 19

<212> PRT

<213> rat

<400> 2

Trp Ile Tyr Pro Gly Asp Gly Asp Thr Asp Tyr Asn Gln Lys Phe Asn

1               5                   10                  15

Gly Lys Ala

<210> 3

<211> 6

<212> PRT

<213> rat

<400> 3

Gly Met Gly Leu Tyr Trp

1               5

<210> 4

<211> 14

<212> PRT

<213> rat

<400> 4

Arg Ser Ser Gln Ser Leu Leu Asp Ser Ala Gly Asn Thr Tyr

1               5                   10

<210> 5

<211> 6

<212> PRT

<213> rat

<400> 5

Leu Val Ser Asn Leu Gly

1               5

<210> 6

<211> 6

<212> PRT

<213> rat

<400> 6

Ser Asn Tyr Gly Met Ala

1               5

<210> 7

<211> 17

<212> PRT

<213> rat

<400> 7

Tyr Ile Ser Tyr Asp Gly Gly Ser Thr Tyr Tyr Arg Asp Ser Val Lys

1               5                   10                  15

Gly

<210> 8

<211> 9

<212> PRT

<213> rat

<400> 8

Thr Pro His Tyr Gly Tyr Lys Tyr Trp

1               5

<210> 9

<211> 13

<212> PRT

<213> rat

<400> 9

Arg Ala Ser Glu Asp Ile Tyr Asn Gly Leu Ala Trp Tyr
1               5                   10

<210> 10
<211> 6
<212> PRT
<213> rat

<400> 10
His Ala Asn Ser Ser His
1               5

<210> 11
<211> 7
<212> PRT
<213> rat

<400> 11
Gln Gln Tyr Tyr Asp Tyr Pro
1               5

<210> 12

<211> 6

<212> PRT

<213> rat

<400> 12

Asn Asp Tyr Tyr Met Ala

1               5

<210> 13

<211> 17

<212> PRT

<213> rat

<400> 13

Thr Ile Ile Tyr Asp Gly Thr Arg Thr Tyr Tyr Arg Asp Ser Val Lys

1               5                   10                  15

Gly

<210> 14

<211> 5

<212> PRT

<213> rat

<400> 14

Thr Pro Trp Gly Ser

1               5

<210> 15

<211> 13

<212> PRT

<213> rat

<400> 15

Arg Ala Ser Gln Gly Ile Ser Asn Phe Leu Asn Trp Tyr

1               5                   10

<210> 16

<211> 5

<212> PRT

<213> rat

<400> 16

Thr Ser Asn Leu Gln

1               5

<210> 17

<211> 8

<212> PRT

<213> rat

<400> 17

Gln Gln Tyr Asp Ser Ser Pro Trp

1               5

<210> 18

<211> 42

<212> DNA

<213> artificial

<220>

<223> antisense primer HAS-1

<400> 18

ccaggggcca gtggatagat agacaagctt gggtgtcgtt tt          42

<210> 19

<211> 38

<212>  DNA

<213>  artificial

<220>

<223>  antisense primer KAS-1

<400>  19

cacgaattct gcagtggcac ctcaggacct ttgggtct                    38

<210>  20

<211>  21

<212>  DNA

<213>  artificial

<220>

<223>  T7 promoter primer

<400>  20

ctaatacgac tcactatagg g                                      21

<210>  21

<211>  19

<212>  DNA

<213>  artificial

<220>

<223>  U-19mer primer

<400>  21

gcccccccag tcacgacgt                                                19

<210>  22

<211>  356

<212>  DNA

<213>  rat

<400>  22

aggtgcagct gcagcagtca ggggctgaac tggtgaagcc tgggtcctca gtgaaaattt      60

cctgcaaggc ttctggctac accttcacca gtaactttat gcactggata aaacagcagc     120

ctggaaatgg ccttgagtgg attgggtgga tttatcctgg agatggtgat acagactata     180

atcaaaagtt caatgggaag gcaacactca ctgcagacaa atcctccagc acagcctata     240

tgcagctcag cagcctaaca tctgaggact ctgcagtcta tttctgtgca agagggatgg     300

gcctttactg gtactttgac ttctggggcc aagggaccac ggtcaccgtc tcctca         356

<210>  23

<211>  342

<212>  DNA

<213>  rat

<220>

<221>  MISC#FEATURE

<222>  (277)..(306)

<223>  n is unknown for frame shift

<400>  23

gacattgtgc taacccaatc tccatccaca ttatcggcta ccattggaca atcggtctcc      60

atctcttgca ggtcaagtca gagtctctta gatagtgctg gaaacaccta tttatattgg     120

tctctacaga ggcctggcca atctccacag cttctaattt atttggtatc caacctggga     180

tctggggtcc ccaacagatt cagtggcagt gggtcaggaa cagatttcac actcaaaatc     240

agtggagtgg aggctgagga tttgggagtt tattacnnnn nnnnnnnnnn nnnnnnnnnn     300

nnnnnnacgt tcggtggagg gaccaagctg gaaataaaac gg                        342

<210>  24

<211>  362

<212>  DNA

<213>  rat

<400>  24

aggtcaagct gcagcagtca gggggcggct tagtgcagcc tggaaggtcc atgaaactct      60

cctgtgcagc ctcaggattc actttcagta actatggcat ggcctgggtc cgccaggctc 120

caaagaaggg tctggagtgg gtcgcataca ttagttatga tggtggtagc acttactatc 180

gagactccgt gaagggccga ttcactatct ccagagataa tgcaaaaagc accctatacc 240

tgcaaatgga cagtctgagg tctgaggaca cggccactta tcactgtaca acaccgcact 300

atgggtataa atactggtac tttgacttct ggggccaagg gaccacggtc accgtctcct 360

ca 362

<210> 25

<211> 321

<212> DNA

<213> rat

<400> 25

gacatccaga tgactcagtc tccagcttcc ctgtctgcat ctctgggaga aactgtcacc 60

atcgaatgtc gagcaagtga ggacatttac aatggtttag catggtatca gcagaagcca 120

gggaaatctc ctcagctcct gatctatcat gcaaatagct cccatactgg ggtcccatca 180

cggttcagcg gcagtggatc tggtacacag tattctctca agatagacag cctgcaatct 240

gaagatgtcg caaattattt ctgtcaacag tattacgatt atcccacgtt tggagctggg 300

accaagctgg agctgaaacg g 321

<210> 26

<211> 337

<212> DNA

<213> rat

<400> 26

aggtccaact gcagcagtca ggcggaggct tagtacagcc tggaaggtcc ctgaatctct 60

cctgtgcagc ctcaggattc actttcaatg actattacat ggcctgggtc cgccaggctc 120

caaaaaaggg tctggagtgg gtcgcaacca ttatttatga tggtactagg acttactatc 180

gagactccgt gaagggccga ttcactatct ccagagataa tgcaaaaagc accctatacc 240

tgcaaatgga cagtctgagg tctgaggaca cggccactta ttactgtgca acaccctggg 300

gctcctgggg ccaagggacc acggtcaccg tctcctc 337

<210> 27

<211> 324

<212> DNA

<213> rat

<400> 27

gacatccaga tgacccagtc tccatcctcc atgcctgcat ctctgggaga gagagtcacc 60

atcagttgca gagcaagtca gggtattagc aattttctaa actggtatca gcagaaagca 120

gatggaacga ttaaacccct gatctactac acatccaatt tacaatctgc tgtcccatca 180

aggttcagtg gcagtgggtc tgggacagat tattctctca ccatcagcag cctggagcct 240

gaagattttg caatgtatta ctgccaacag tatgatagtt ctccgtggac gttcggtgga 300

ggcaccaagc tggaaatcac acgg 324

<210> 28

<211> 119

<212> PRT

<213> rat

<400> 28

Glu Val Gln Leu Gln Gln Ser Gly Ala Glu Leu Val Lys Pro Gly Ser

1               5                   10                  15

Ser Val Lys Ile Ser Cys Lys Ala Ser Gly Tyr Thr Phe Thr Ser Asn

            20                  25                  30

Phe Met His Trp Ile Lys Gln Gln Pro Gly Asn Gly Leu Glu Trp Ile

        35                  40                  45

Gly Trp Ile Tyr Pro Gly Asp Gly Asp Thr Asp Tyr Asn Gln Lys Phe

    50                  55                  60

Asn Gly Lys Ala Thr Leu Thr Ala Asp Lys Ser Ser Ser Thr Ala Tyr

65                  70                  75                  80

Met Gln Leu Ser Ser Leu Thr Ser Glu Asp Ser Ala Val Tyr Phe Cys

                85                  90                  95

Ala Arg Gly Met Gly Leu Tyr Trp Tyr Phe Asp Phe Trp Gly Gln Gly

            100                 105                 110

Thr Thr Val Thr Val Ser Ser

        115

<210> 29

<211> 114

<212> PRT

<213> rat

<220>

<221> MISC#FEATURE

<222> (93)..(102)

<223> Xaa is unknown amino acid

<400> 29

Asp Ile Val Leu Thr Gln Ser Pro Ser Thr Leu Ser Ala Thr Ile Gly

1               5                   10                  15

Gln Ser Val Ser Ile Ser Cys Arg Ser Ser Gln Ser Leu Leu Asp Ser

            20                  25                  30

Ala Gly Asn Thr Tyr Leu Tyr Trp Ser Leu Gln Arg Pro Gly Gln Ser

        35                  40                  45

Pro Gln Leu Leu Ile Tyr Leu Val Ser Asn Leu Gly Ser Gly Val Pro

    50                  55                  60

Asn Arg Phe Ser Gly Ser Gly Ser Gly Thr Asp Phe Thr Leu Lys Ile

65                  70                  75                  80

Ser Gly Val Glu Ala Glu Asp Leu Gly Val Tyr Tyr Xaa Xaa Xaa Xaa

                85                  90                  95

Xaa Xaa Xaa Xaa Xaa Xaa Thr Phe Gly Gly Gly Thr Lys Leu Glu Ile

            100                 105                 110

Lys Arg

<210>  30

<211>  121

<212>  PRT

<213>  rat

<400>  30

Glu Val Lys Leu Gln Gln Ser Gly Gly Gly Leu Val Gln Pro Gly Arg

1               5                   10                  15

Ser Met Lys Leu Ser Cys Ala Ala Ser Gly Phe Thr Phe Ser Asn Tyr

            20                  25                  30

Gly Met Ala Trp Val Arg Gln Ala Pro Lys Lys Gly Leu Glu Trp Val

        35                  40                  45

Ala Tyr Ile Ser Tyr Asp Gly Gly Ser Thr Tyr Tyr Arg Asp Ser Val

    50                  55                  60

Lys Gly Arg Phe Thr Ile Ser Arg Asp Asn Ala Lys Ser Thr Leu Tyr
65                  70                  75                  80
Leu Gln Met Asp Ser Leu Arg Ser Glu Asp Thr Ala Thr Tyr His Cys
                85                  90                  95
Thr Thr Pro His Tyr Gly Tyr Lys Tyr Trp Tyr Phe Asp Phe Trp Gly
            100                 105                 110
Gln Gly Thr Thr Val Thr Val Ser Ser
        115                 120

<210>  31
<211>  107
<212>  PRT
<213>  rat

<400>  31
Asp Ile Gln Met Thr Gln Ser Pro Ala Ser Leu Ser Ala Ser Leu Gly
1               5                   10                  15
Glu Thr Val Thr Ile Glu Cys Arg Ala Ser Glu Asp Ile Tyr Asn Gly
            20                  25                  30
Leu Ala Trp Tyr Gln Gln Lys Pro Gly Lys Ser Pro Gln Leu Leu Ile
        35                  40                  45
Tyr His Ala Asn Ser Ser His Thr Gly Val Pro Ser Arg Phe Ser Gly

    50                  55                  60

Ser Gly Ser Gly Thr Gln Tyr Ser Leu Lys Ile Asp Ser Leu Gln Ser

65                  70                  75                  80

Glu Asp Val Ala Asn Tyr Phe Cys Gln Gln Tyr Tyr Asp Tyr Pro Thr

                85                  90                  95

Phe Gly Ala Gly Thr Lys Leu Glu Leu Lys Arg

            100                 105

<210> 32

<211> 113

<212> PRT

<213> rat

<400> 32

Glu Val Gln Leu Gln Gln Ser Gly Gly Gly Leu Val Gln Pro Gly Arg

1               5                   10                  15

Ser Leu Asn Leu Ser Cys Ala Ala Ser Gly Phe Thr Phe Asn Asp Tyr

            20                  25                  30

Tyr Met Ala Trp Val Arg Gln Ala Pro Lys Lys Gly Leu Glu Trp Val

        35                  40                  45

Ala Thr Ile Ile Tyr Asp Gly Thr Arg Thr Tyr Tyr Arg Asp Ser Val

    50                  55                  60

Lys Gly Arg Phe Thr Ile Ser Arg Asp Asn Ala Lys Ser Thr Leu Tyr
65                  70                  75                  80
Leu Gln Met Asp Ser Leu Arg Ser Glu Asp Thr Ala Thr Tyr Tyr Cys
                85                  90                  95
Ala Thr Pro Trp Gly Ser Trp Gly Gln Gly Thr Thr Val Thr Val Ser
            100                 105                 110
Ser

<210>  33
<211>  108
<212>  PRT
<213>  rat

<400>  33
Asp Ile Gln Met Thr Gln Ser Pro Ser Ser Met Pro Ala Ser Leu Gly
1               5                   10                  15
Glu Arg Val Thr Ile Ser Cys Arg Ala Ser Gln Gly Ile Ser Asn Phe
            20                  25                  30
Leu Asn Trp Tyr Gln Gln Lys Ala Asp Gly Thr Ile Lys Pro Leu Ile
        35                  40                  45
Tyr Tyr Thr Ser Asn Leu Gln Ser Ala Val Pro Ser Arg Phe Ser Gly
    50                  55                  60

Ser Gly Ser Gly Thr Asp Tyr Ser Leu Thr Ile Ser Ser Leu Glu Pro
65                  70                  75                  80
Glu Asp Phe Ala Met Tyr Tyr Cys Gln Gln Tyr Asp Ser Ser Pro Trp
                85                  90                  95
Thr Phe Gly Gly Gly Thr Lys Leu Glu Ile Thr Arg
            100                 105